# EUROPEAN PATENT SPECIFICATION

(11) **EP 3 773 274 B1**
(45) Date of publication and mention of the grant of the patent: **28.05.2025**
(21) Application number: 19785220.5
(22) Date of filing: 12.04.2019
(51) Int. Cl.: A61B 17/34, A61M 5/46, A61M 39/02, A61M 39/04, A61M 5/31, A61M 39/00

(54) **LOW-PROFILE VASCULAR ACCESS DEVICE**
FLACHE GEFÄSSZUGANGSVORRICHTUNG
DISPOSITIF D'ACCÈS VASCULAIRE À PROFIL BAS

(30) Priority: 13.04.2018 US 201862657662 P; 18.09.2018 US 201862732928 P; 11.04.2019 US 201916382177
(43) Date of publication of application: 17.02.2021
(73) Proprietor: C. R. Bard, Inc., Franklin Lakes, NJ 07417 (US)
(72) Inventor: FEDOR, Brenda L.F., Holladay, UT 84117 (US); STATS, Jason R., Layton, UT 84041 (US); RANDALL, Michael Adam, Gilbert, AZ 85298 (US); VAN LIERE, Chad C., Phoenix, AZ 85042 (US); COX, Jeremy B., Salt Lake City, UT 84109 (US)
(74) Representative: Hoffmann Eitle
(86) International application number: PCT/US2019/027301
(87) International publication number: WO 2019/200304

(56) References cited:
- WO-A1-96/29112
- WO-A1-99/42166
- WO-A2-01/80926
- US-A- 4 695 273
- US-A- 4 929 236
- US-A- 5 084 015
- US-A- 5 281 199
- US-A- 5 281 199
- US-A- 5 399 168
- US-A- 5 741 228
- US-A- 5 848 989
- US-A1- 2005 171 502
- US-A1- 2008 009 832
- US-A1- 2009 118 683
- US-A1- 2012 184 925
- US-A1- 2012 209 221
- US-A1- 2015 196 704
- US-A1- 2016 158 493
- US-A1- 2018 078 751
- US-A1- 2018 078 751

## Description

### STATE OF THE ART

State of the art that may be beneficial for understanding the present invention can be found in the following documents:
WO 01/80926 A2 relates to a hemodialysis port, comprising a flexible housing member defining one or more ports. US 5 281 199 relates to an access port for implantation within the body of a patient for providing repeated access to specific tissue within the patient and communicating with the tissue by an implanted internal catheter. WO 99/42166 A1 relates to a vascular access port having a housing that includes a base and a cap that capture compound septum to seal each of a pair of distinct fluid reservoirs in the base. US 5 848 989 A relates to an implantable port having a low profile housing for delivery/collection of fluids into and out of a body. US 5 084 015 A relates to a catheter assembly of the hypodermic embedment type for infusing a fluid medicament such as a carcinostatic agent into a body cavity such as a blood vessel for therapeutic purposes. US 2018/078751 A1 relates to a low-profile access port for subcutaneous implantation within a patient. US 2015/196704 A1 relates to a subcutaneous implantable vascular access port that reduces the risk of infection by creating a larger surface area for needle access with multiple palpable windows.

### BRIEF SUMMARY

The present invention is defined by the appended independent claims. The dependent claims are directed to optional features and preferred examples.
In aspects of the present invention, there are provided devices according to the independent claims.
Preferred examples are set out in the dependent claims and in the remaining part of the description.

Briefly summarized, embodiments of the present invention are directed to a low-profile access port for subcutaneous implantation within the body of a patient. The access port includes a receiving cup that provides a relatively large subcutaneous target to enable a catheter-bearing needle to access the port without difficulty. In addition, the access port includes a valve/seal assembly to permit pressurized fluid injection through the port while preventing backflow.

In an aspect of the invention a device is provided that allows immediate subcutaneous dialysis access while allowing patients to bathe and shower. Such a device reduces costs and time associated with cleaning and maintenance relative to traditional tunneled dialysis catheter positioned external to the body.

In an aspect of the invention, a device is provided enabling long-term dialysis while minimizing skin trauma. Typical infusion or apheresis port interfaces forces a clinician to access the approximately the same locus every time the port is accessed. Dialysis is typically required multiple times per week. Embodiments of an implantable dialysis port is provided that allows for multiple needle insertion sites, thereby reducing trauma to a single locus on the skin.

In an aspect of the invention, a low-profile access port comprises a body including a conduit with an inlet port at a proximal end thereof, and a receiving cup. The receiving cup is concavely shaped to direct a catheter-bearing needle into the conduit via the inlet port. The receiving cup is oriented substantially toward a skin surface when subcutaneously implanted within the patient to ease needle impingement thereon. A valve/seal assembly disposed in the conduit enables passage of the catheter therethrough while preventing fluid backflow.

In an aspect of the invention, a low-profile access port for subcutaneous implantation within the patient is disclosed and comprises a body including a conduit with an inlet port at a proximal end thereof, and a receiving cup. The receiving cup is funnel shaped to direct a catheter-bearing needle into the conduit via the inlet port. The conduit is defined by the body and extends from the inlet port to an outlet defined by a stem. A bend in the conduit enables catheter advancement past the bend while preventing needle advancement. A valve/seal assembly is also disposed in the conduit and enables passage of the catheter therethrough while preventing fluid backflow. The body includes radiopaque indicia configured to enable identification of the access port via x-ray imaging.

In an aspect of the invention, a low-profile access port is disclosed and comprises a body including a first set of receiving cups, a first set of inlet ports, each receiving cup of the first set of receiving cups in fluid communication with an inlet port of the first set of inlet ports, each receiving cup concavely shaped to direct an impinging needle toward the inlet port. A first conduit in fluid communication with each inlet port of the first set of inlet ports, the first conduit extending from the first set of inlet ports to a first outlet of a port stem and a catheter in fluid communication with the first outlet.
A second set of receiving cups are in fluid communication with an inlet port of the second set of inlet ports, and a second conduit in fluid communication with each inlet port of the second set of inlet ports, the second conduit extending from the second set of inlet ports to a second outlet of the port stem.

In some embodiments, the first set of receiving cups may be proximal to the second set of receiving cups. A perimeter of each receiving cup of the first set of receiving cups lies in a plane, and wherein the plane of the perimeter of each receiving cup is angled with respect to one another. A perimeter of each receiving cup of the first set of receiving cups lies in a plane, and wherein the plane of the perimeter of each receiving cup is co-planar with respect to one another. A perimeter of each receiving cup of the first set of receiving cups includes a cutout, the cutout between adjacent receiving cups providing communication therebetween.

In an aspect of the invention, a vascular access device for subcutaneous implantation is disclosed and comprises a catheter having a first lumen and a second lumen, an elongate body defining a first elongate chamber and a second elongate chamber, the first elongate chamber in fluid communication with the first lumen and the second elongate chamber in fluid communication with the second lumen. A needle penetrable septum is disposed over an opening in an upper surface of the elongate body, the opening providing access to the first elongate chamber and the second elongate chamber. A needle impenetrable guide disposed over the opening and the needle penetrable septum, the needle impenetrable guide including a plurality of first openings positioned over the first elongate chamber, and a plurality of second openings positioned over the second elongate chamber.

In some embodiments, the elongate body has a length and a width, the length more than two times greater than the width. The first elongate chamber and the second elongate chamber extend in a side-by-side arrangement relative to a longitudinal axis of the elongate body. The first elongate chamber and the second elongate chamber are in a tandem arrangement relative to a longitudinal axis of the elongate body such that the first elongate chamber is proximal to the second elongate chamber. The impenetrable needle guide is disposed at least partially within the needle penetrable septum. The impenetrable needle guide does not penetrate the needle penetrable septum. The plurality of first openings are parallel to the plurality of second openings.

In light of the above, embodiments herein are generally directed to a vascular access device, also referred to herein as an access port, for subcutaneous implantation within the body of a patient. The implanted access port is transcutaneously accessible by a catheter-bearing needle, such as a peripheral intravenous ("PIV") catheter, so as to place the PIV catheter into fluid communication with the access port. A fluid outlet of the access port is operably connected to an in-dwelling catheter disposed within the vasculature of a patient, in one embodiment, to enable the infusion into and/or removal of fluids from the patient's vasculature to take place via the PIV catheter.

These and other features of embodiments of the present invention will become more fully apparent from the following description and appended claims, or may be learned by the practice of embodiment of the invention as set forth hereinafter.

### BRIEF DESCRIPTION OF THE DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIGS. 1A-1E show various views of an access port according to one example useful for understanding the invention;
FIG. 2 is a cross sectional view of the access port of FIGS. 1A-1E;
FIG. 3A-3C are various views of a low-profile access port according to one example useful for understanding the invention;
FIG. 4 is a top view of a low-profile access port according to one example useful for understanding the invention;
FIG. 5 is a perspective view of a low-profile access port according to one example useful for understanding the invention
FIG. 6 is a perspective view of a low-profile access port according to one example useful for understanding the invention;
FIGS. 7A and 7B are various views of an access port according to one example useful for understanding the invention;
FIGS. 8A and 8B are various views of an access port according to one example useful for understanding the invention;
FIGS. 9A-9G depict various views of a low-profile vascular access device according to one example useful for understanding the invention;
FIG. 10 is an exploded view of the access device of FIGS. 9A-9G;
FIG. 11 is a cross-sectional view of the access device of FIGS. 9A-9G;
FIGS. 12A-12C depict various views of a seal according to one example useful for understanding the invention;
FIGS. 13A-13C depict various views of a valve according to one example useful for understanding the invention;
FIGS. 14A-14D depict various stages of insertion of a catheter into the access device of FIGS. 9A-9G;
FIGS. 15A and 15B depict various views of a guide device for use with the access device of FIGS. 9A-9G according to one example useful for understanding the invention;
FIGS. 16A-16G depict various views of a low-profile vascular access device according to one example useful for understanding the invention;
FIGS. 17A and 17B depict various views of the vascular access port of FIGS. 16A-16G;
FIG. 18 is an exploded view of the vascular access device of FIGS. 16A-16G;
FIG. 19 is a partially transparent view of the vascular access device of FIGS. 16A-16G;
FIG. 20 is a perspective view of a portion of the vascular access device of FIGS. 16A-16G;
FIGS. 21A and 21B are cutaway views of the vascular access device of FIGS. 16A-16G;
FIGS. 22A-22C depict various views of a low-profile vascular access device according to one example useful for understanding the invention;
FIG. 23 is a partially transparent view of the vascular access device of FIGS. 22A-22C;
FIG. 24 is a partially transparent view of a portion of the vascular access port of FIGS. 22A-22C;
FIGS. 25A-25E depict various views of a low-profile vascular access device according to one example useful for understanding the invention;
FIGS. 26A-26D depict various views of a low-profile vascular access device according to one example useful for understanding the invention;
FIG. 27 is a cross-sectional view of a valve/seal configuration according to one example useful for understanding the invention;
FIG. 28 is a cross-sectional view of a valve/seal configuration according to one example useful for understanding the invention;
FIG. 29 is a cross-sectional view of a valve/seal configuration according to one example useful for understanding the invention;
FIG. 30 is a cross-sectional view of a valve/seal configuration according to one example useful for understanding the invention;
FIG. 31 is a perspective view of a portion of a vascular access device according to one example useful for understanding the invention;
FIGS. 32A-32B depict perspective views of a low-profile vascular access device according to one embodiment;
FIG. 33 depict a perspective view of a low-profile vascular access device according to one embodiment;
FIGS. 34A-34B depict perspective views of a low-profile vascular access device according to one embodiment;
FIGS. 35A-35J depict various views of a low-profile vascular access device according to one embodiment;
FIG. 36A depict a perspective view of a low-profile vascular access device according to one embodiment;
FIG. 36B depicts an exploded view of a low-profile vascular access device according to one embodiment;
FIG. 37A depicts a perspective view of a port assembly of the device of FIG. 36A;
FIG. 37B depicts an exploded view of the port assembly of FIG. 37A;
FIGS. 38A-38B depict various views of the catheter of the device of FIG. 36A; and
FIG. 39A-39C depict various views of the connector of the device of FIG. 36A.

### DETAILED DESCRIPTION OF SELECTED EMBODIMENTS

Reference will now be made to figures wherein like structures will be provided with like reference designations. It is understood that the drawings are diagrammatic and schematic representations of exemplary embodiments of the present invention, and are neither limiting nor necessarily drawn to scale.

For clarity it is to be understood that the word "proximal" refers to a direction relatively closer to a clinician using the device to be described herein, while the word "distal" refers to a direction relatively further from the clinician. For example, the end of a catheter placed within the body of a patient is considered a distal end of the catheter, while the catheter end remaining outside the body is a proximal end of the catheter. Also, the words "including," "has," and "having," as used herein, including the claims, shall have the same meaning as the word "comprising."

Embodiments of the present invention are generally directed to an access port for subcutaneous implantation within the body of a patient. The implanted access port is transcutaneously accessible by a catheter-bearing needle, such as a peripheral intravenous ("PIV") catheter, so as to place the PIV catheter into fluid communication with the access port. A fluid outlet of the access port is operably connected to an in-dwelling catheter disposed within the vasculature of a patient, in one embodiment, to enable the infusion into and/or removal of fluids from the patient's vasculature to take place via the PIV catheter, e.g. dialysis or similar extracorporeal treatment.

In accordance with one embodiment, the access port defines a low profile so as to facilitate ease of placement within the subcutaneous tissue of the patient. Further, the access port is configured to provide a relatively large subcutaneous target to enable the PIV catheter or other suitable catheter-bearing needle to access the port without difficulty. In addition, the access port includes a valve/seal assembly to permit the injection of fluids through the access port at a relatively high flow rate, such as about 5 ml per second at a pressure of about 300 psi (also referred to herein as "power injection"). Possible applications for the access port described herein include administration of medicaments and other fluids to the patient, pheresis/apheresis/dialysis or similar extracorporeal treatments that enable fluid to be infused into or removed from the patient's vasculature , fluid aspiration, etc.

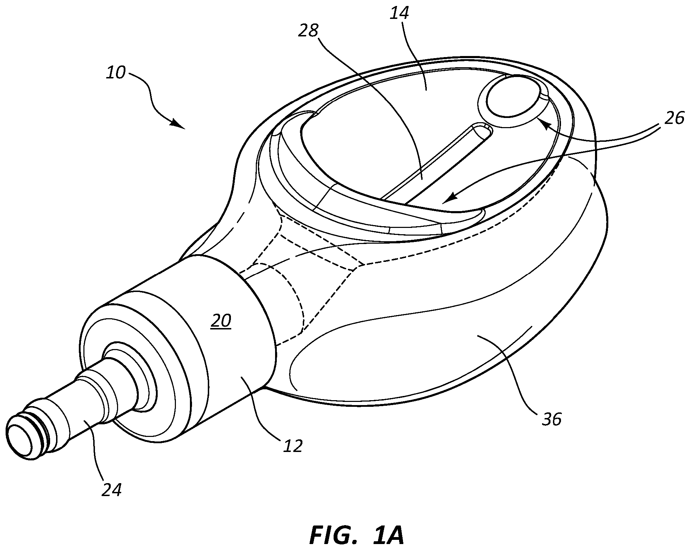

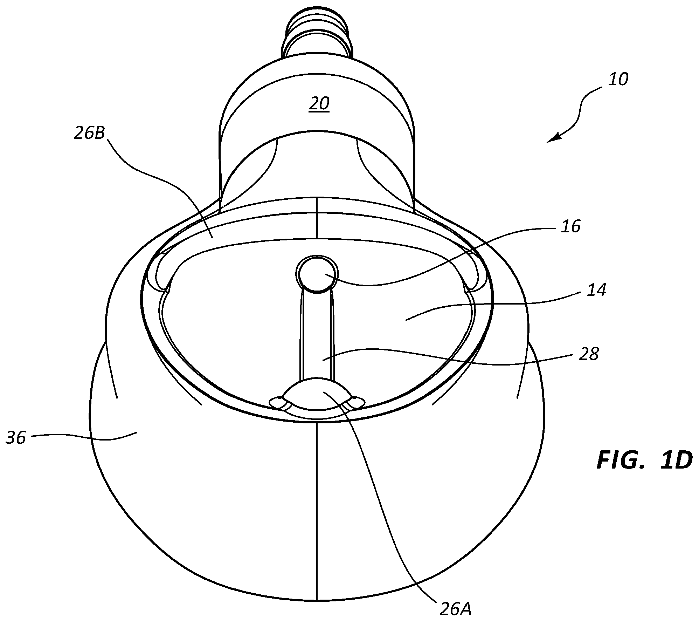

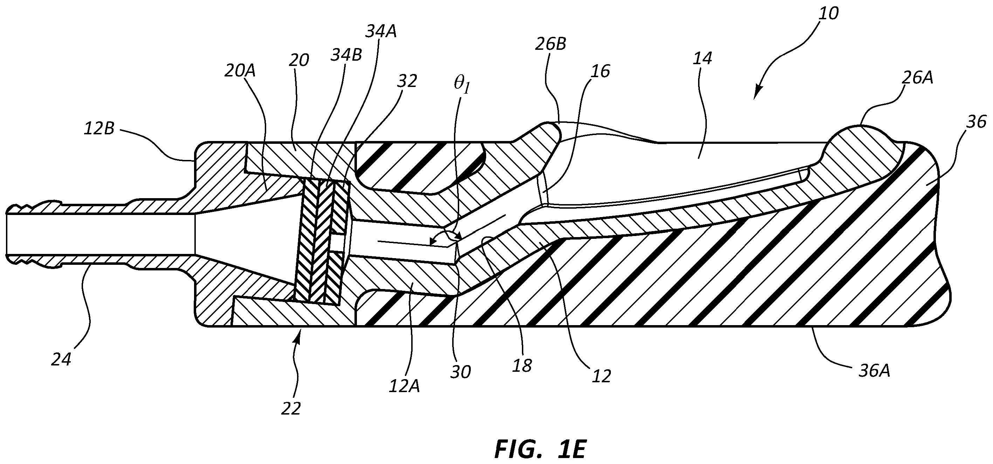

Reference is first made to FIGS. 1A-1E, which show various details of an access port, generally designated at 10, in accordance with one example useful for understanding the invention. As shown, the port 10 includes a body 12 that is defined in the present example by a first portion 12A and a second portion 12B (FIG. 1E). In the present example of the port body 12 includes a metal such as titanium, and as such, the second portion 12B is press fit into engagement with the first portion 12A to define the body, though it is appreciated that the port body can include a variety of other materials, including metals, thermoplastics, ceramics, etc.

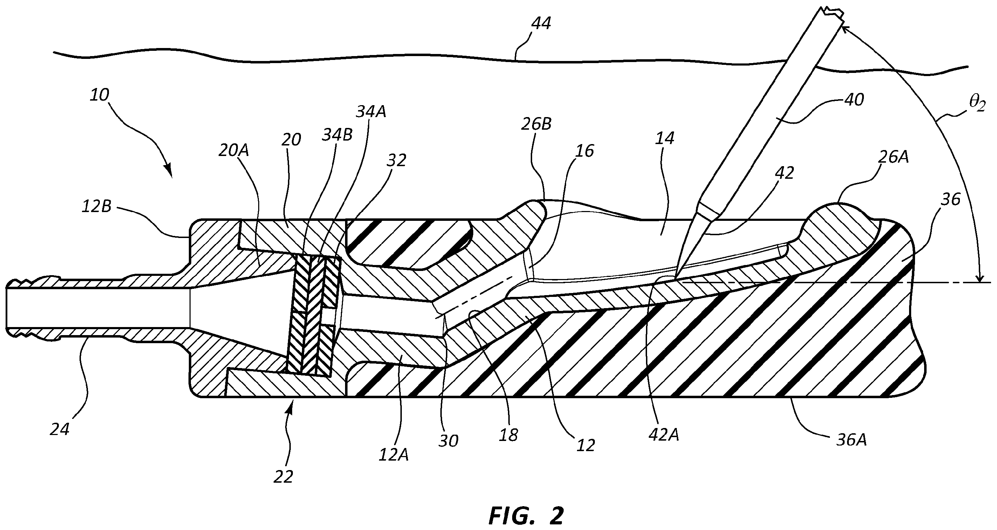

The port body 12 defines in the present example a substantially concavely-shaped receiving cup 14 for receiving and directing a catheter-bearing needle (FIG. 2) to operably connect with the port 10, as described further below. In particular, the substantially concave shape of the receiving cup 14 is configured to direct a catheter-bearing needle (FIG. 2) impinging thereon toward an inlet port 16 that serves as an opening for a conduit 18 defined by the port body 12. The open and shallow nature of the receiving cup 14 together with its substantially upward orientation (*i.e.,* toward the skin surface of the patient), so that it is substantially parallel to the skin surface when subcutaneously implanted under the skin of the patient (*i.e.,* the receiving cup is substantially parallel to the skin surface when the skin is at rest, or undeformed by digital pressure or manipulation), enables the receiving cup to present a large, easily accessible target for the needle when introduced into the skin, as seen in FIG. 2. FIG. 2 further shows that the port 10 defines a relatively low profile height, which enables relatively shorter needle lengths to be used for accessing the port after implantation. It will be appreciated that the port 10, port body 12, funnel 14, portions thereof, or the like, can be constructed of a suitable biocompatible material. Further, the port 10, or portions thereof can include metals, for example titanium. Such metals can be biocompatible, radiopaque, and/or resistant to gouging from an impinging needle, as will be discussed in more detail herein. By way of example, the port 10, port body 12, funnel 14, portions thereof that include titanium, can be machined, can be formed by injection-molding powdered titanium, or manufactured via other suitable methods.

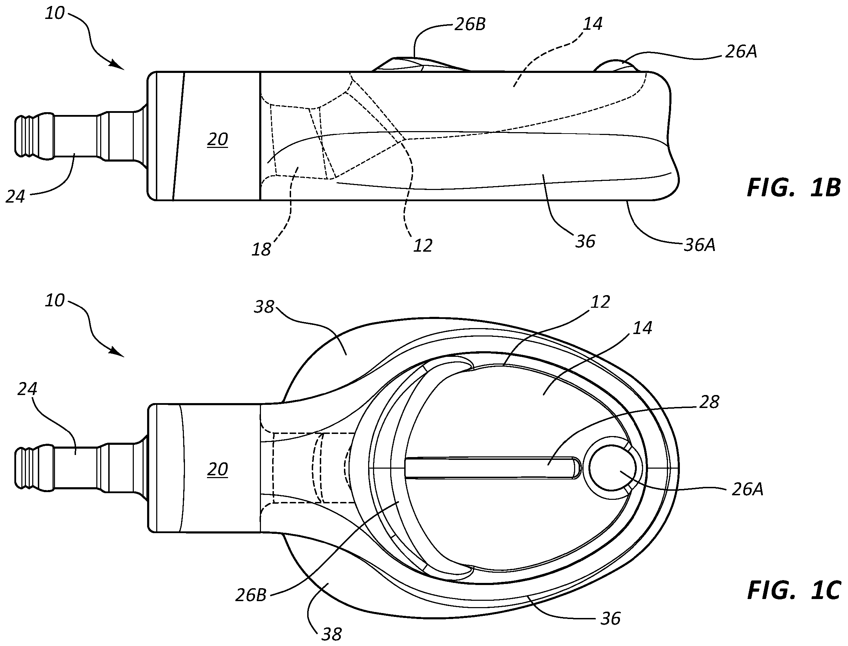

Palpation features 26 are included with the port body 12 to assist a clinician to locate and/or identify the port 10 via finger palpation after implantation under the skin of the patient. In detail, the palpation features 26 in the present example include a bump 26A disposed near the proximal end of the receiving cup 14 and a ridge 26B disposed above and curving around a distal portion of the receiving cup. FIG. 1B shows that the palpation features extend above the general upper plane defined by the port 10 so as to facilitate palpation of the features by a clinician in order to locate the position and/or orientation of the receiving cup 14. Note that a variety of other sizes, configurations, numbers, etc., of palpation features can be included on the port in addition to what is shown and described herein.

A guide groove 28 is defined on the receiving cup 14 and is longitudinally aligned with the inlet port 16 of the conduit 18. The guide groove 28 is defined as a depression with respect to adjacent portions of the surface of the receiving cup 14 and extends distally along the receiving cup surface from a proximal portion of the receiving cup so as to provide a guide path to guide the distal tip of the catheter-bearing needle toward the inlet port 16 once impingement of the needle into the guide groove is made. This in turn reduces the chance the needle will slide across and off the receiving cup 14 during insertion. Note that these and other similar features, though differing in shape and configuration, can also be included on the other ports disclosed herein.

As best seen in FIG. 1E, the port body 12 further defines the conduit 18 as a pathway into which a transcutaneously inserted catheter can pass so as to place the catheter in fluid communication with the port 10. As shown, the conduit 18 is in communication with the receiving cup 14 via the inlet port 16. A first conduit portion 18A of the conduit 18 distally extends from the inlet port 16 in an angled downward direction from the perspective shown in FIG. 1E to a bend 30, where a second conduit portion 18B of the conduit angles slightly upward and changes direction at a predetermined angle θ₁. Note that angle orientation θ₁ in one example is about 37 degrees, but can vary from this in other examples, including angles less than 37 degrees in one example. The magnitude of angle θ₁ depends in one example on various factors, including the size of the catheter and/or needle to be inserted into the port conduit, the size of the conduit itself, etc.

The conduit 18 then extends to and through a cavity 20A defined by a valve housing 20 of the port body. The conduit 18 extends to a distal open end of the stem 24 of the port 10. The conduit 18 is sized so as to enable the catheter 40 (FIG. 2) to pass therethrough, as will be seen.

As mentioned, the valve housing 20 defines a cavity 20A through which the conduit passes and which houses a valve/seal assembly 22. The valve/seal assembly 22 includes a sealing element, or seal 32, which defines a central hole through which the catheter 40 can pass, a first slit valve 34A and a second slit valve 34B. The seal 32 and valves 34A, 34B are sandwiched together in one example and secured in place within the cavity 20A as shown in FIG. 1E. The slits of the slit valves 34A, 34B are rotationally offset from one another by about 90 degrees in the present example, though other relationships are possible.

The seal 32 and valves 34A, 34B of the valve/seal assembly 22 cooperate to enable fluid-tight passage therethrough of the catheter 40 (FIG. 2) while also preventing backflow of fluid through the valve/seal assembly. Indeed, in one example the seals disclosed herein prevent fluid flow around the external portion of the catheter when the catheter is disposed through the seal, while the valves are suitable for preventing fluid flow when no catheter passes through them. As such, when the catheter 40 is not inserted therethrough the valve/seal assembly 22 seals to prevent passage of air or fluid. In the present example, the seal 32 and valves 34A, 34B include silicone, though other suitably compliant materials can be employed.

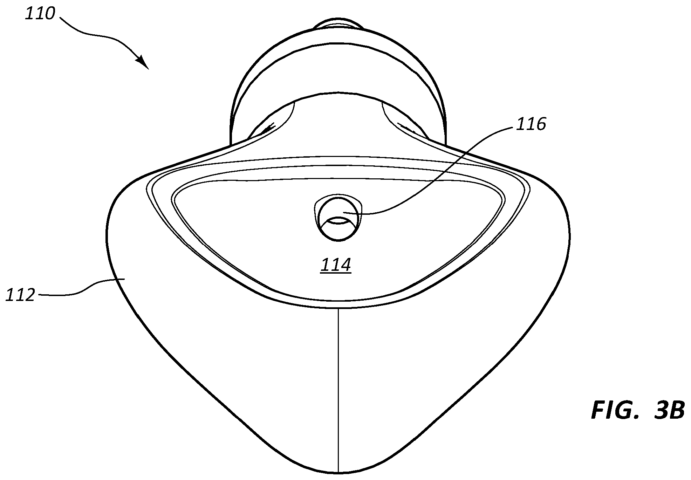

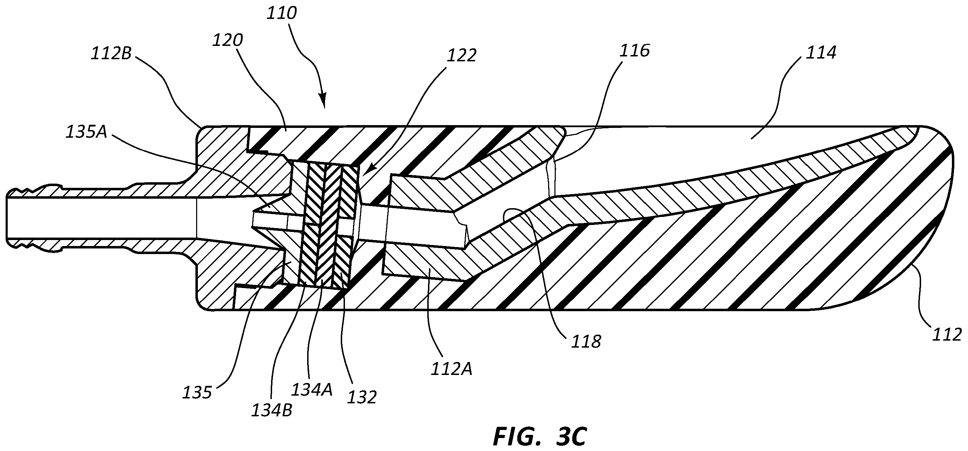

The port 10 in the present example includes an overmolded portion 36 that covers the port body 12. The overmolded portion 36 includes silicone or other suitably compliant material and surrounds the body 12 as shown so as to provide a relatively soft surface for the port 10 and reduce patient discomfort after port implantation. The overmolded portion 36 includes two predetermined suture locations 38, best seen in FIG. 1C, for suturing the port 10 to patient tissue, though sutures may be passed through other portions of the overmolded portion, if desired. The overmolded portion 36 further defines a relatively flat bottom surface 36A so as to provide a stable surface for the port 10 in its position within the tissue pocket after implantation. In contrast, the port shown in FIG. 3C includes a bottom surface with a slightly rounded profile.

FIG. 2 depicts details regarding the insertion of the catheter 40 disposed on the needle 42, according to one example useful for understanding the invention. After locating the port 10 via through-skin palpation of the palpation features 26, a clinician uses the catheter-bearing needle 42 to pierce a skin surface 44 and insert the needle until a distal tip 42A thereof impinges on a portion of the receiving cup 14, as shown. Note that, because of the orientation of the receiving cup 14 as substantially parallel to the skin surface, the needle 42 can impinge on the receiving cup at an insertion angle θ₂ that is relatively steep, which facilitates ease of needle insertion into the body. Indeed, in one example a needle inserted substantially orthogonally through the skin of the patient can impinge the receiving cup of the access port.

The needle 42 is manipulated until the distal tip 42A is received into the guide groove 28, which will enable the distal tip to be guided along the groove to the inlet port 16. The needle 42 is then inserted through the inlet port 16 and into the first portion 18A of the conduit 18 until it is stopped by the bend 30. The needle 42 can then be proximally backed out a small distance, and the catheter 40 advanced over the needle such that the catheter bends and advances past the bend 30 into the second portion 18B of the conduit 18. Catheter advancement continues such that a distal end 40A of the catheter 40 advances into and past the hole of the seal 32 and through both slits of the slit valves 34A, 34B of the valve/seal assembly 40. Once the distal end 40A of the catheter 40 has extended distally past the valve/seal assembly 22, further advancement can cease and fluid transfer through the catheter 40 and port 10 can commence, including infusion and/or aspiration through the stem 24. Once fluid transfer is completed, the catheter 40 can be withdrawn proximally through the valve/seal assembly 22 and the conduit, then withdrawn through the surface 44 of the skin and out of the patient.

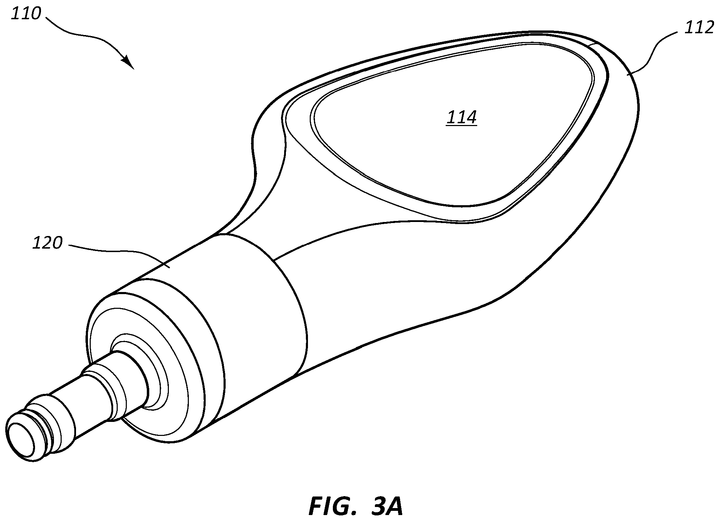

FIGS. 3A-3C depict details of an access port 110 according to another example. Note that various similarities exist between the port 10 and the other ports shown and described herein. As such, only selected port aspects are discussed below. As shown, the port 110 includes a body 112 that in turn includes a first body portion 112A and a second body portion 112B, best seen in FIG. 3C. The body 112 in the present example includes a thermoplastic, such as an acetyl resin in the present example. As such, the first and second body portions 112A, 112B are ultrasonically welded to one another to define the body 12, in the present example. As before, a receiving cup 114 is included with the body 112 and is operably connected to a conduit 118 via an inlet port 116. Also, note that a variety of materials can be used to define the port body, receiving cup, conduit, etc.

A valve/seal assembly 122 is disposed within a cavity 120A that is defined by a valve housing 120, which in the present example, is defined by the first body portion 112A. The valve/seal assembly 122 includes a proximal seal 132 with a central hole for catheter passage, two slit valves 134A, 134B each with a slit arranged at a 90-degree offset with respect to the other, and a distal seal 135 with a central hole, also referred to herein as a sphincter seal.

The distal seal 135 includes on its distal surface a frustoconical portion 135A disposed about the seal central hole that is configured to provide a sphincter-like seal about the outer surface of a catheter when it extends through the valve/seal assembly. The frustoconical portion 135A is disposed such that any back-flowing fluid impinging on the frustoconical portion will cause the seal to secure itself about the outer surface of the catheter in an even tighter engagement, thus preventing backflow past the catheter outer surface when high fluid pressures are present, such as in the case of power injection. As mentioned, other valve/seal combinations can also be included in the valve/seal assembly.

In the present example, the receiving cup 114 and portion of the conduit 118 proximal to the valve/seal assembly 122 both include a needle-impenetrable lining that prevents the distal end of a needle from gouging the surface when impinging thereon. This, in turn, prevents the undesirable creation of material flecks dug by the needle. Various suitable materials can be employed for the needle-impenetrable material, including glass, ceramic, metals, etc. In one example, the components of the port 110 are all non-metallic such that the port is considered MRI-safe, by which the port does not produce undesired artifacts in MRI images taken of the patient when the port is in implanted therewithin.

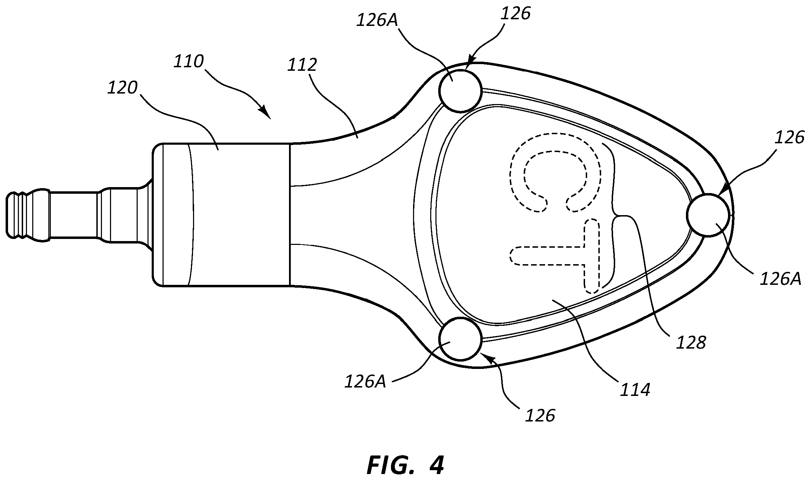

FIG. 4 depicts additional features of the port 110 according to another example useful for understanding the invention. As shown, in the present example the receiving cup 18 includes radiopaque indicia 128 to indicate a characteristic of the port 110. Here, the radiopaque indicia 128 includes a "C" and a "T" that are formed by a radiopaque material, such as tungsten, bismuth trioxide, etc., so as to be visible after port implantation via x-ray imaging technology. For instance, the radiopaque material can be formed as an insert that is insert-molded included in the port body, as an initially flowable material that is injected into a cavity of the port body before hardening, etc. In examples where the port body is metallic, the radiopaque indicia can be formed by etching, engraving, or otherwise producing a relative thickness difference between the indicia and the surrounding port body material so as to produce an x-ray-discernible contrast that shows up in an x-ray image.

In the present example, the CT radiopaque indicia 128 indicate to an observer that the port is capable of power injection of fluids therethrough. In addition to this characteristic, other characteristics can be indicated by various other types of indicia as appreciated by one skilled in the art.

Further, in the present example the top view of the port 110 of FIG. 4 indicates that the port body 112 in the region surrounding the receiving cup 114 defines a generally triangular shape, which can be palpated by a clinician after implantation and can indicate not only the location of the receiving cup, but also a particular characteristic of the port, such as its ability to be used for power injection. Of course, the receiving cup may define shapes other than triangular in other examples.

FIG. 4 further shows that distributed about the perimeter of the receiving cup 114 are three palpation features 126, namely, three suture plugs 126A disposed in corresponding holes defined in the port body 112. The suture plugs 126A include raised silicone bumps in the present example and can serve to locate the position of the receiving cup 114 post-implantation when they are palpated by a clinician prior to needle insertion into the patient. Various other palpation features could be included with the port, in other embodiments.

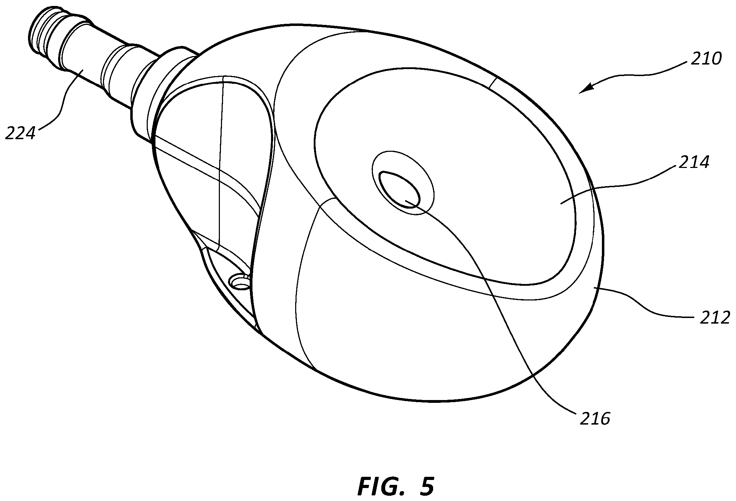

FIG. 5 depicts details of a low-profile port 210 according to one example useful for understanding the invention, including a body 212 defining a concavely-shaped receiving cup 214 and an inlet port 216 positioned slightly off-center with respect to the receiving cup. A stem 224 is included as a fluid outlet.

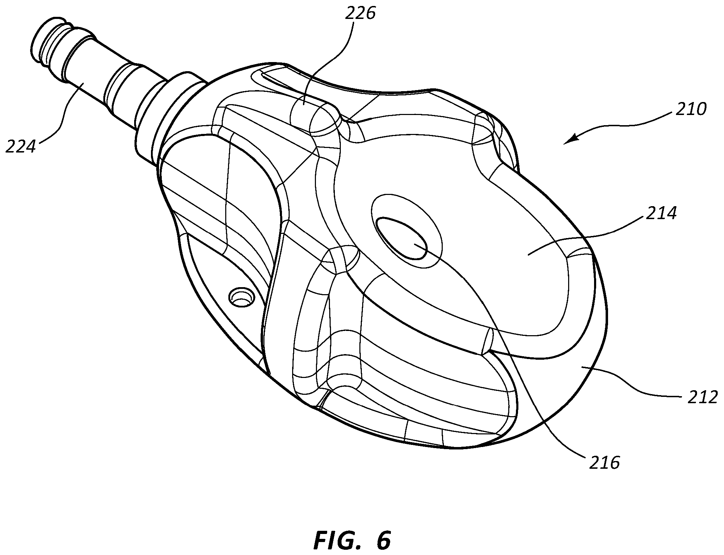

FIG. 6 depicts the low-profile port 210 according to another example useful for understanding the invention, wherein the body 212 defining additional surface features, including a raised palpation feature 226 distal to the receiving cup 214. In light of FIGS. 5 and 6, it is thus appreciated that the port can be configured in a variety of shapes and configurations to provide a low-profile solution for providing vascular access. Note also that the receiving cup shape, design, and configuration can vary from is explicitly shown and described herein.

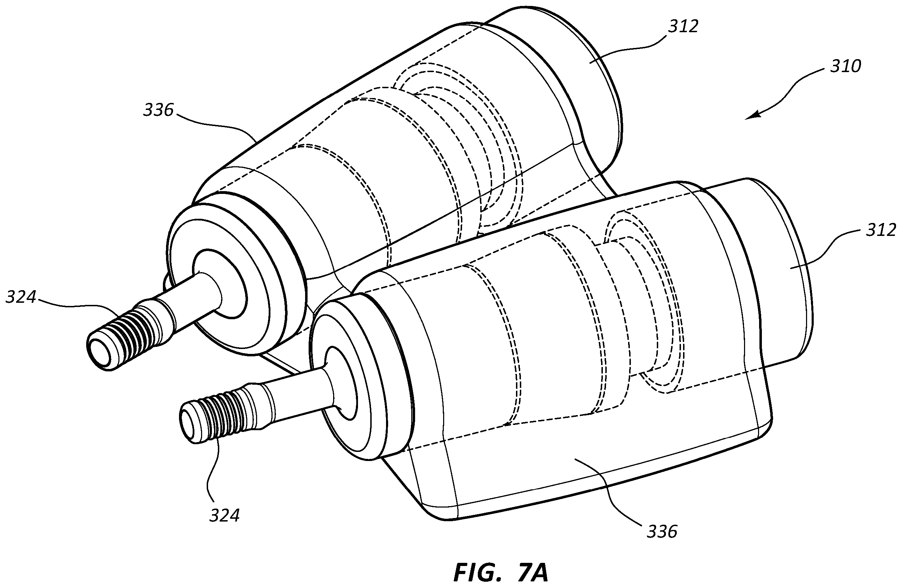

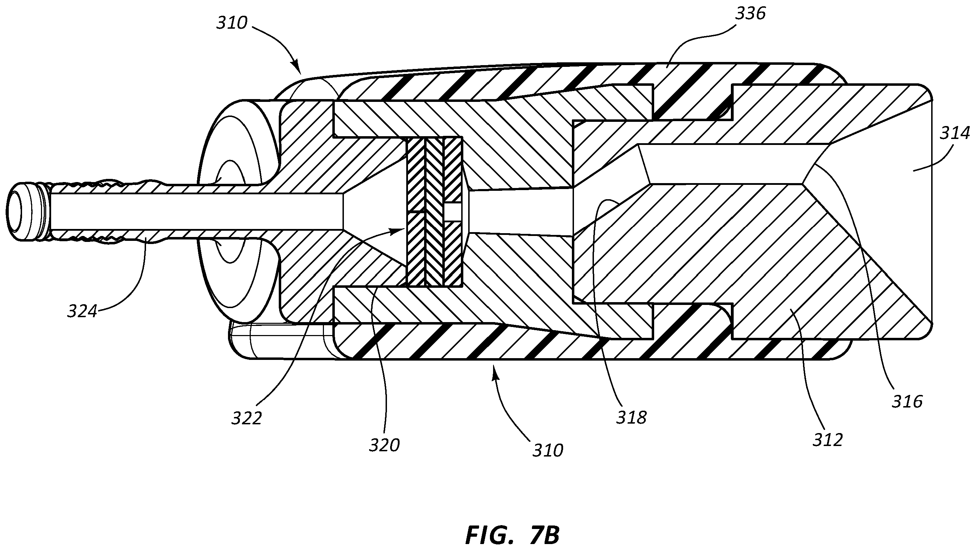

FIGS. 7A and 7B depict various details of a low-profile dual-body access port 310 according to one example useful for understanding the invention, wherein each of the port bodies 312 defines a receiving cup 314 that is laterally facing and includes an inlet port 316 leading to a conduit 318. The conduit 318 extends distally to a valve/seal assembly 322 disposed in a valve housing 320, which in the present example, is defined by a portion of the body 312. The conduit 318 extends through the port 324. A compliant overmolded portion 324 covers portions of each body 312 of the port 310 and operably joins the bodies to one another. The bodies 312 can include any suitable material, including metal, thermoplastic, etc.

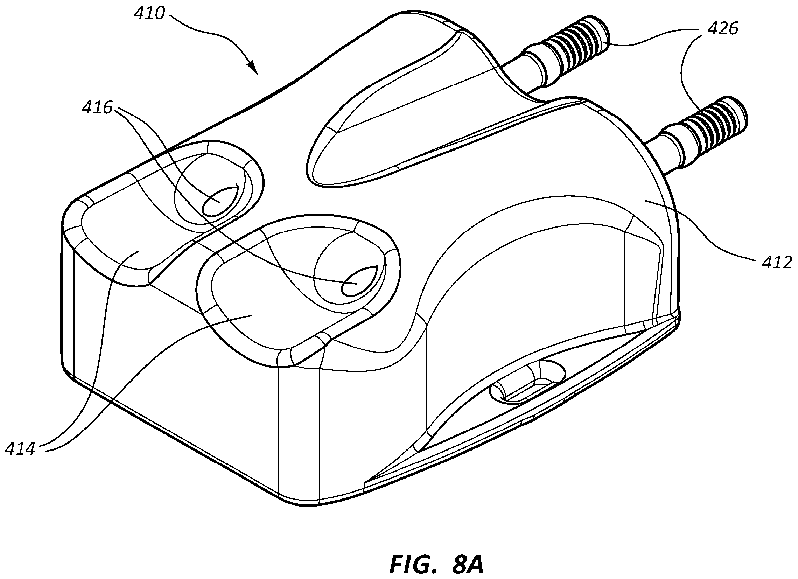

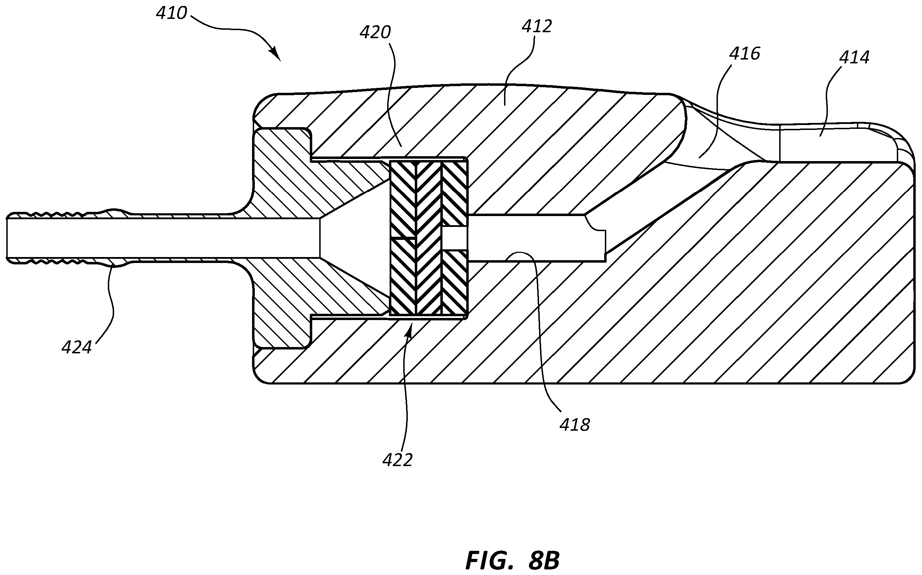

FIGS. 8A and 8B depict various details of a low-profile dual-body access port 410 according to one example useful for understanding the invention, wherein a port body 412 defines dual fluid paths. Each fluid path includes a receiving cup 414 defined by the body 412 and facing a substantially upward orientation from the perspective shown in FIGS. 8A and 8B. An inlet port 416 is included with each receiving cup 414 and defines the opening to a conduit 418. Each conduit 418 extends distally to a valve/seal assembly 422 disposed in a valve housing 420, which in the present example, is defined by a portion of the body 412. The conduit 418 extends through the port 424. The body 412 can include any suitable material, including metal, thermoplastic, etc.

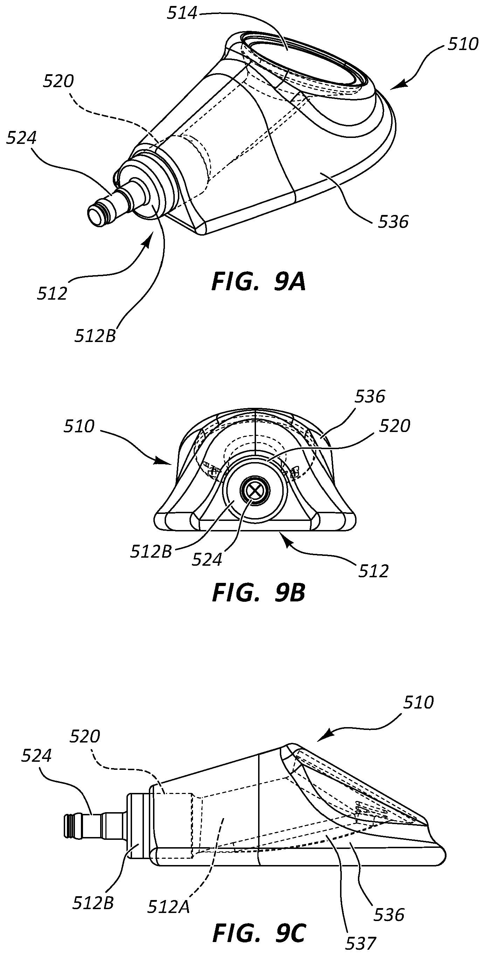

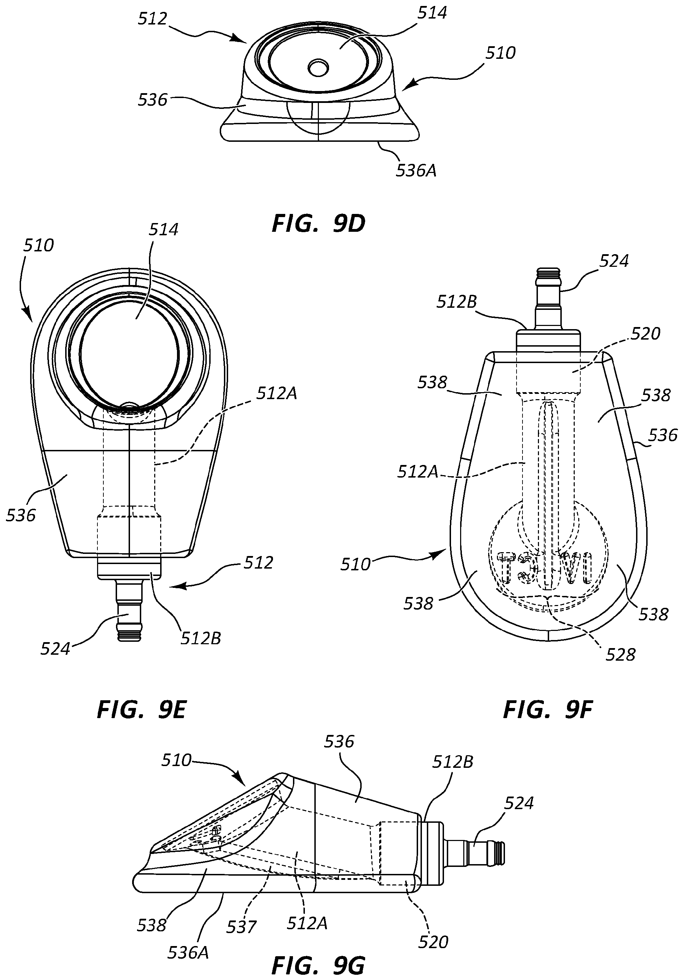

Reference is now made to FIGS. 9A-30, which depict various details of examples useful for understanding the invention generally directed to vascular access devices, also referred to herein as access ports, for subcutaneous implantation within the body of a patient. The implanted access ports to be described are transcutaneously accessible by a catheter-bearing needle, such as a peripheral intravenous ("PIV") catheter, so as to place the PIV catheter into fluid communication with the access port. A fluid outlet of the access port is operably connected to an in-dwelling catheter disposed within the vasculature of a patient, in one example, to enable the infusion into and/or removal of fluids from the patient's vasculature to take place via the PIV catheter.

In accordance with one example, the access port defines a relatively low profile so as to facilitate ease of placement within the subcutaneous tissue of the patient. Further, the access port is configured to provide a relatively large subcutaneous target to enable the PIV catheter or other suitable catheter-bearing needle to access the port without difficulty. In addition, the access port includes a valve/seal assembly to permit power injection of fluids through the access port. As before, possible applications for the access port described herein include administration of medicaments and other fluids to the patient, pheresis/apheresis, fluid aspiration, etc.

Reference is first made to FIGS. 9A-9G, which show various details of a vascular access device (also "access port" or "port"), generally designated at 510, in accordance with one example useful for understanding the invention. As shown, the port 510 includes a body 512 that is defined in the present example by a first portion 512A and a second portion 512B (FIG. 9E). In the present example the port body 512 includes a metal such as titanium, and as such, the second portion 512B is press fit into engagement with the first portion 512A to define the body, though it is appreciated that the port body can include a variety of other materials, including metals, thermoplastics, ceramics, etc.

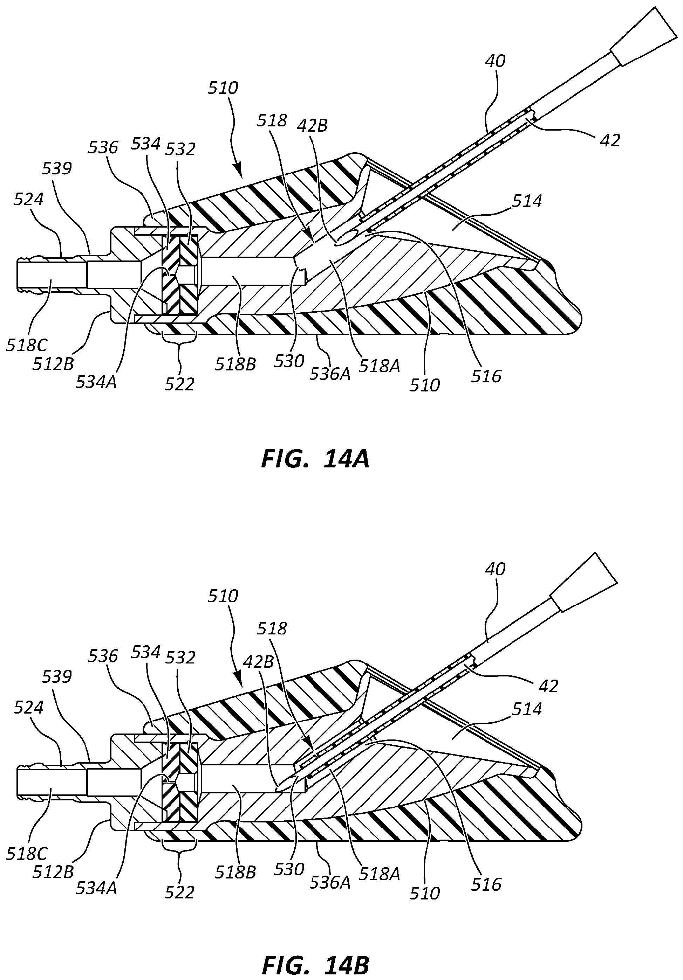

The port body first portion 512A defines in the present example a substantially funnel-shaped receiving cup 514 for receiving and directing a catheter-bearing needle (FIG. 14A) to operably connect with the port 510, as described further below. In particular, the substantially funnel shape of the receiving cup 514 is configured to direct the catheter-bearing needle (FIG. 14A) impinging thereon toward an inlet port 516 that serves as an opening for a conduit 518 defined by the port body 512. The open and shallow nature of the receiving cup 514, angled toward the skin surface of the patient enables the receiving cup to present a large, easily accessible target for the needle when introduced into the skin, as seen in FIGS. 14A-14D. FIGS. 9B and 9C further show that the port 510 defines a relatively low profile height, which enables relatively shorter needle lengths to be used for accessing the port after implantation. Note that palpation features can be included with the port body 512 to assist a clinician to locate and/or identify the port 510 via finger palpation after implantation under the skin of the patient, as with other examples herein. Further, in another example a guide groove can be defined on the receiving cup 514 to be longitudinally aligned with the inlet port 516 of the conduit 518, similar to that shown in the access port 10 of FIG. 1A.

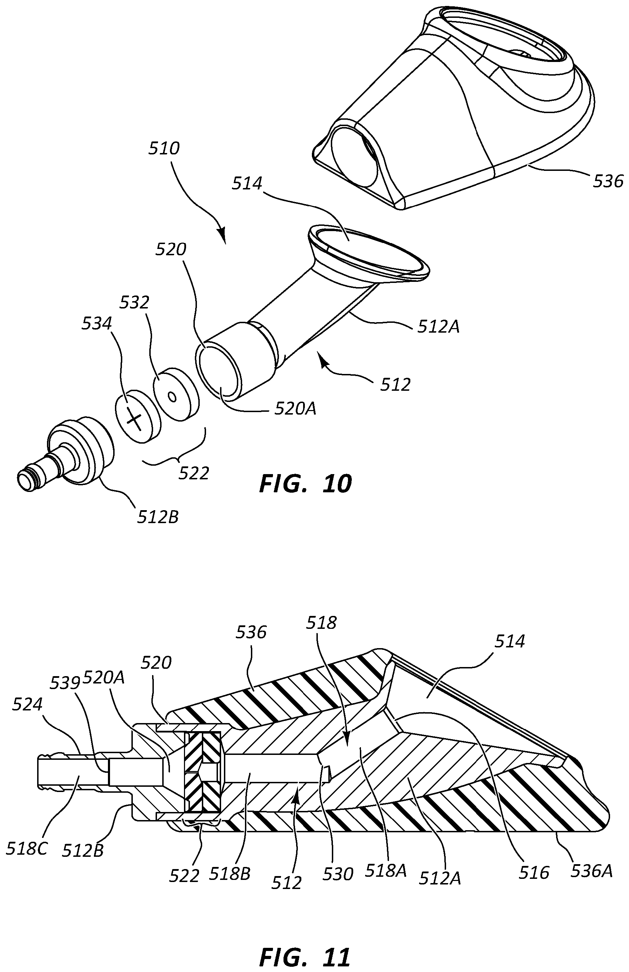

Together with FIGS. 9A-9G, reference is also made to FIGS. 10 and 11. As best seen in FIG. 11, the port body 512 further defines the conduit 518 as a pathway into which a transcutaneously inserted catheter can pass so as to place the catheter in fluid communication with the port 510 and the indwelling catheter attached to the stem 524 thereof. As shown, the conduit 518 is in fluid communication with the receiving cup 514 via the inlet port 516. A first conduit portion 518A of the conduit 518 distally extends from the inlet port 516 in an angled downward direction from the perspective shown in FIG. 11 to a bend 530, where a second conduit portion 518B of the conduit extends substantially horizontally (from the perspective shown in FIG. 11) at a predetermined angle with respect to the first conduit portion. Note that predetermined angle at the bend 530 in one example is about 34 degrees, but can vary from this in other examples, including angles less or more than 34 degrees in one example. The magnitude of the predetermined angle at the bend 530 depends in one example on various factors, including the size of the catheter and/or needle to be inserted into the port conduit, the size of the conduit itself, etc.

The conduit 518 then extends to and through a cavity 520A defined by a valve housing 520 of the port body 12 where a third conduit portion 518C extends to a distal open end of the stem 524 of the port 510. In the present example the conduit 518 is sized so as to enable the catheter 40 (FIG. 14A) to pass therethrough to a predetermined point, as will be seen.

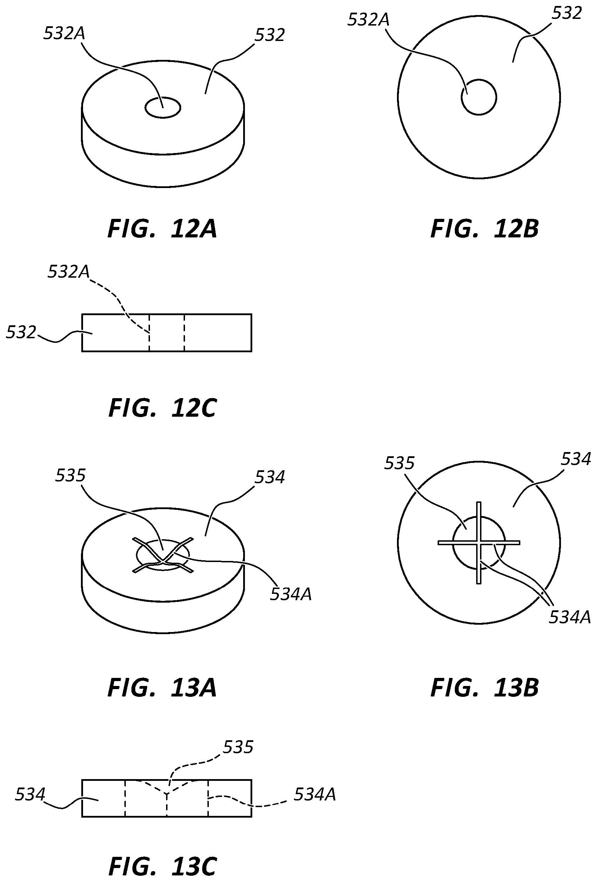

As mentioned, the valve housing 520, defined by portions of the first and second portions 512A, 512B of the body 512 defines a cavity 520A through which the conduit 518 passes and which houses a valve/seal assembly 522. The valve/seal assembly 522 includes a sealing element, or seal 532, which defines a central hole 532A (FIGS. 12A-12C) through which the catheter 40 (FIG. 14A) can pass, and a slit valve 534 including two intersecting slits 534A (FIGS. 13A-13C). The seal 532 and valve 534 are sandwiched together in one example, with the seal 532 disposed proximal to the valve 534, and secured in place within the cavity 520A as shown in FIG. 11. The slits 534A of the slit valve 534 are orthogonally offset from one another by about 90 degrees in the present example, though other relationships are possible. Note that the valve 534 includes a central depression 535 to ease the transition of passage of the catheter 40 from the seal 532 to the valve.

The seal 532 and valve 534 of the valve/seal assembly 522 cooperate to enable fluid-tight passage therethrough of the catheter 40 (FIG. 14A) while also preventing backflow of fluid through the valve/seal assembly. Indeed, in one example the seals disclosed herein prevent fluid flow around the external portion of the catheter when the catheter is disposed through the seal 532, while the valve 534 is suitable for preventing fluid flow when no catheter passes through them. As such, when the catheter 40 is not inserted therethrough the valve/seal assembly 522 seals to prevent passage of air or fluid through the conduit 518. In the present example, the seal 532 and valve 534 are composed of silicone, such as SILASTIC^{®} Q7-4850 liquid silicone rubber available from Dow Corning Corporation, though other suitably compliant materials can be employed. In one example, silicone oil, such as NuSil Technology Med 400 silicone oil, is included with the seal 532 and valve 534 to enhance lubricity and extend component life. In another example, the silicone oil is infused into the silicone.

The port 510 in the present example includes an overmolded portion 536 that covers a majority portion of the port body 512. The overmolded portion 536 includes silicone, such as SILASTIC^{®} Q7-4850 liquid silicone rubber or other suitably compliant material and surrounds the body 512 as shown so as to provide a relatively soft surface for the port 510 and reduce patient discomfort after port implantation within the patient body. The overmolded portion 536 includes in one example predetermined suture locations 538, best seen in FIG. 9F, for suturing the port 510 to patient tissue, though sutures may be passed through other portions of the overmolded portion, if desired. The overmolded portion 536 further defines a relatively flat bottom surface 536A so as to provide a stable surface for the port 510 in its position within the tissue pocket after implantation into the patient body.

FIGS. 9C and 9G show that the first body portion 512A defines a securement ridge 537 that serves as an anchor to prevent relative movement between the overmolded portion 536 and the body 512. The securement ridge 537 can vary in shape, number, configuration, etc. Note that the overmolded portion 536 in one example is molded in a molding process over the body 512. In another example, the overmolded portion 536 is separately formed then adhesively attached to the body 512, such as via Med A adhesive. These and other configurations are therefore contemplated.

FIGS. 14A-14D depict details regarding the insertion of the catheter 40 disposed on the needle 42 into the port 510 (already subcutaneously implanted into the body of the patient), according to one example useful for understanding the invention. After locating the port 510 (optionally via through-skin palpation of palpation features, such as a top portion of the overmolded portion 536 and/or the receiving cup 514), a clinician uses the catheter-bearing needle 42 to pierce a skin surface and insert the needle until a distal tip 42B thereof impinges on a portion of the receiving cup 514, as shown in FIG. 14A. Note that, because of the orientation of the receiving cup 514 is angled substantially toward the skin surface, the needle 42 can impinge on the receiving cup at an insertion angle that is relatively steep, which facilitates ease of needle insertion into the body. Indeed, in one example a needle inserted substantially orthogonally through the skin of the patient can impinge the receiving cup of the access port. In another, example, the insertion angle of the needle 42 can be relatively shallow, similar to current insertion angles for IV catheters.

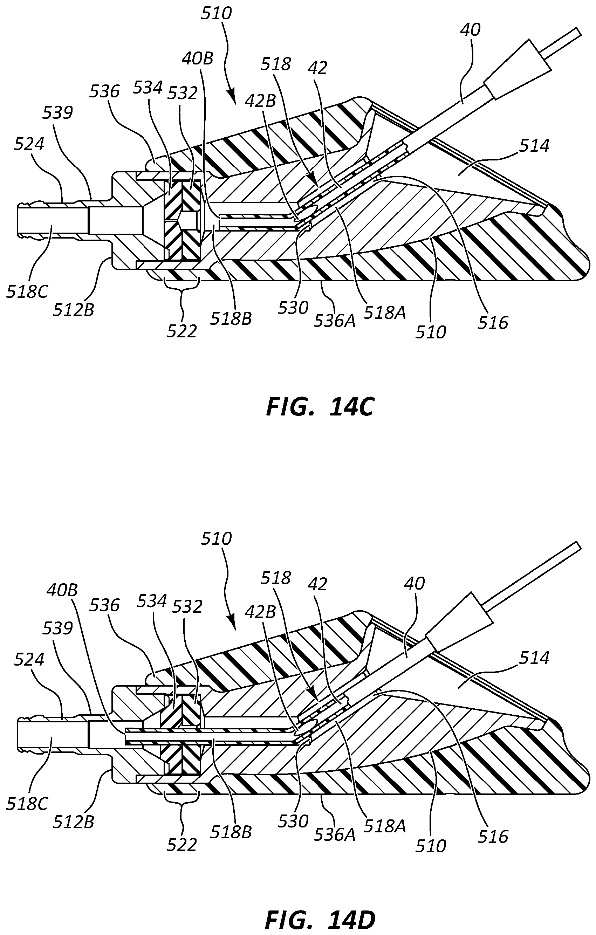

The needle 42 is manipulated by the clinician and guided by impingement on the receiving cup 514 until the needle distal tip 42B is guided to the inlet port 516. The needle 42 is then inserted through the inlet port 516 and into the first portion 518A of the conduit 518 until it is stopped by the bend 530, as seen in FIG. 14B. The needle 42 can then be proximally backed out a small distance, and the catheter 40 advanced over the needle such that the catheter bends and advances past the bend 530 into the second portion 518B of the conduit 518, as seen in FIG. 14C. Catheter advancement continues such that a distal end 40B of the catheter 40 advances into and past the hole 532A of the seal 532 and through both slits 534A of the slit valve 534 of the valve/seal assembly 522. Note that the length of the second conduit portion 518B is sufficient to enable the cross-sectional shape of the distal portion of the catheter 40 to return to a substantially round shape from the oval shape imposed thereon as a result of its passage through the conduit bend 530.

Once the distal end 40B of the catheter 40 has extended distally past the valve/seal assembly 522, further advancement is prevented by impingement of the catheter distal end against an annular stop surface 539 included in the third conduit portion 518C defined by the stem 524, as shown in FIG. 14D and in more detail in FIG. 11. In one example, the stop surface 539 is defined as an annular shoulder and is sized so as to stop advancement of one size of catheter, such as 14 Gauge catheter, while allowing a 16 Gauge catheter to pass. In another example, no stop surface is included in the conduit 518, thus enabling the catheter 40 to advance completely past the distal end of the stem 524, if desired. Note that the port conduit can be configured to accept one or more of a variety of catheter Gauge sizes, including 14 Gauge, 16 Gauge, 18 Gauge, etc.

Once the catheter 40 is positioned as shown in FIG. 14D, the needle 42 can be fully removed and fluid transfer through the catheter 40 and port 510 can commence, including infusion and/or aspiration through an indwelling catheter attached to the stem 524. (Note that the needle 42 can be removed at another stage of the catheter insertion procedure, in one example.) Dressing of the catheter 40 can also occur as needed. Once fluid transfer is completed, the catheter 40 can be withdrawn proximally through the valve/seal assembly 522 and the conduit 518, then withdrawn through the surface of the skin and out of the patient.

FIG. 9F depicts that, in the present example, the receiving cup 514 includes radiopaque indicia 528 to indicate a characteristic of the port 510. Here, the radiopaque indicia 528 includes an "IVCT" alphanumeric designation that is defined as a depression or recess into the titanium material forming the first body portion 512A so as to be visible after port implantation via x-ray imaging technology. The "IVCT" designation indicates that the port 510 is configured for power injection and is further configured to receive therein a peripheral IV catheter.

In another example the radiopaque indicia 528 can be included by employing radiopaque material that can be formed as an insert that is insert-molded included in the port body, such as an initially flowable material that is injected into a cavity of the port body before hardening, etc. In examples where the port body is metallic, the radiopaque indicia can be formed by metal injection molding, machining, etching, engraving, or otherwise producing a relative thickness difference between the indicia and the surrounding port body material so as to produce an x-ray-discernible contrast that shows up in an x-ray image, similar to FIG. 1F.

In addition to above designation, other characteristics can be indicated by various other types of radiopaque indicia as appreciated by one skilled in the art.

As in other examples described herein, in one example the perimeter of the receiving cup (or other suitable location) can include palpation features, such as three raised bumps in the overmolded portion 536 to assist in locating the position of the receiving cup 514 post-implantation when they are palpated by a clinician prior to needle insertion into the patient. Various other palpation features could be included with the port, in other examples, including disposal on the receiving cup itself, etc.

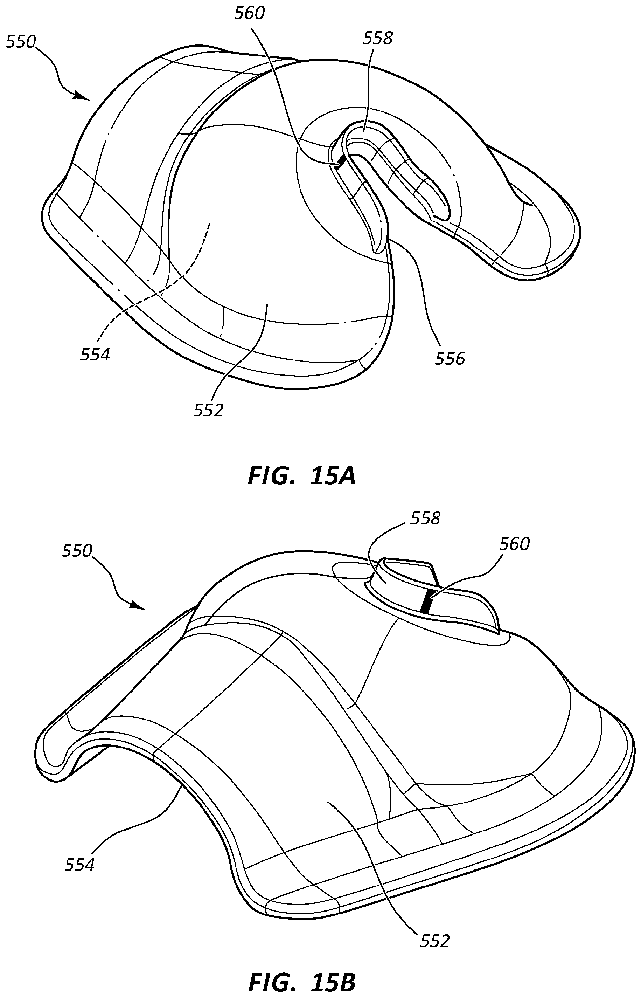

FIGS. 15A and 15B depict details of a guide device 550 that can be placed on the patient skin atop the implanted location of the port 510 shown in FIGS. 9A-9G to assist in guiding the needle 42 through the skin so as to impinge on the receiving cup 514, as desired. As shown, the guide device 550 includes a body 552 that defines a cavity 554 into which a portion of the subcutaneous implanted port 510 will reside when the guide device is pressed on the skin over the port. A notch 556 is included on the body 552, partially bordered by a ridge 558. The notch 556 enables the needle 42 to be passed therethrough so as to be inserted through the skin and into port 510. A marker line 560 is included on the ridge 548 to assist the clinician in placing the needle 42 at the proper orientation and location for impingement on the receiving cup 514, as desired. Note that the shape, size, and other configuration of the guide device can vary from what is shown and described herein.

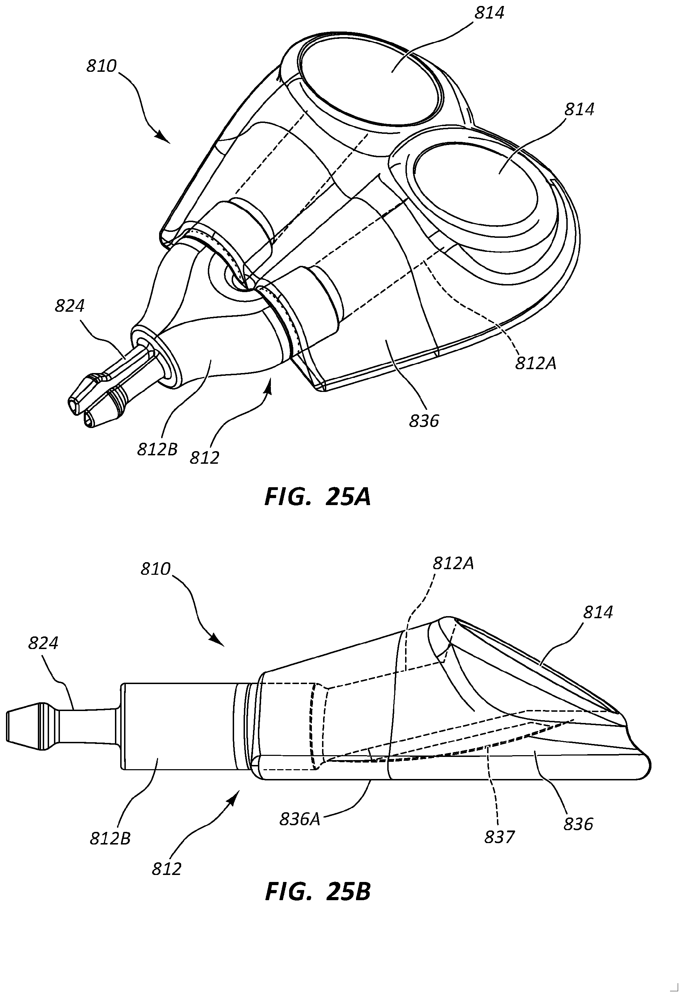

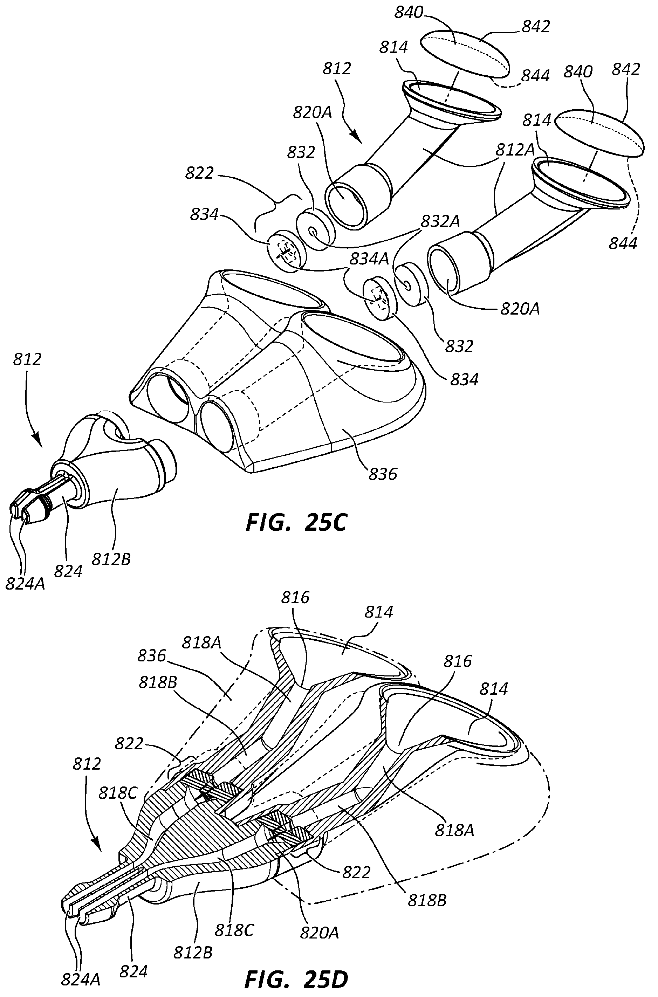

Reference is now made to FIGS. 25A-25E, which show various details of a dual-lumen vascular access device, generally designated at 810, in accordance with one example useful for understanding the invention. As shown, the port 810 includes a body 812 that is defined in the present example by two similarly shaped portions: a single first portion 812A and a single second portion 812B (FIG. 25C). In the present example the port body first and second portions 812A, 812B include a metal such as titanium, and as such, the second portion is press fit into engagement with the first portion to define the body, though it is appreciated that the port body can include a variety of other materials, including metals, thermoplastics, ceramics, etc., and can include other joining methods including adhesive, ultrasonic or other welding, interference fit, etc.

Both port body first portions 812A define in the present example a substantially funnel-shaped receiving cup 814 for receiving and directing the catheter-bearing needle 42 (FIG. 14A) to operably connect with the port 810 in a manner similar to that already described above. In particular, the substantially funnel shape of each receiving cup 814 is configured to direct the catheter-bearing needle 42 impinging thereon toward an inlet port 816 that serves as an opening for a respective conduit 818 defined by the port body 812. The open and shallow nature of each receiving cup 814, angled toward the skin surface of the patient enables the receiving cup to present a large, easily accessible target for the needle when introduced into the skin and directed toward the subcutaneously implanted access port 810. FIG. 25B further shows that the access port 810 defines a relatively low profile height, which enables relatively shorter needle lengths to be used for accessing the subcutaneous access port after implantation.

Note that, as already mentioned, palpation features can be included with the port body 812 in one example to assist a clinician to locate and/or identify the port 810 via finger palpation after implantation under the skin of the patient. Note that a variety of sizes, configurations, numbers, etc., of palpation features can be included on the port. In another example, a guide groove can be defined on the receiving cup 814 to be longitudinally aligned with the inlet port 816 of the conduit 818, as discussed in connection with the example of FIGS. 1A-2. The guide groove can be defined as a depression with respect to adjacent portions of the surface of the receiving cup 814 and extend distally along the receiving cup surface from a proximal portion of the receiving cup so as to provide a guide path to guide the distal tip of the catheter-bearing needle toward the inlet port 816 once impingement of the needle into the guide groove is made. This in turn reduces the chance the needle will slide across and off the receiving cup 814 during insertion. Note that these and other similar features, though differing in shape and configuration, can also be included on the other ports disclosed herein.

In an example, the receiving cup 814 is covered by a septum 840. The septum 840 can be a self-sealing, needle penetrable septum, capable of receiving multiple needle piercings to allow access to the receiving cup 814 there below. Accordingly, the septum 840 can be made of a suitable needle-penetrable material, such as silicone, or the like. The septum 840 includes an outer surface 842 and an inner surface 844 opposite that of the outer surface 842 and substantially facing receiving cup 814. Either of the outer or inner surfaces 842, 844 can be flat or slightly convex. In an example, the inner surface 844 is substantially flat while the outer surface 842 is convex to align with the rounded outer surface of the overmolded portion 836 and provide a continuous outer profile to the port 810. Advantageously, the septum 840 completes a convexly rounded outer profile to the port 810 that allows for a smooth implantation of the device within a tissue pocket and reduces patient discomfort after port implantation within the patient body. Further the septum 840 can prevent tissue ingrowth into the receiving cup 814, and associated conduits 818, that would otherwise obstruct the path of the needle entering the device. Accordingly, the septum 840 prevents additional surgeries required to remove such obstructions or to replace the device 810 prematurely. It will be appreciated that septum 840 can also be applied to any example disclosed herein.

As best seen in FIG. 25D, the port body 812 further defines the two conduits 818, each conduit serving as a pathway into which a transcutaneously inserted catheter can be partially inserted so as to place the catheter in fluid communication both with the port 810 and an indwelling dual-lumen catheter operably attached to two fluid outlets 824A of a stem 824 of the port. As shown, the conduit 818 of each port body first portion 812A is in fluid communication with its respective receiving cup 814 via the inlet port 816. A first conduit portion 818A of the conduit 818 distally extends from the inlet port 816 in an angled downward direction from the perspective shown in FIG. 25D to a conduit bend 830, where a second conduit portion 818B of the conduit extends at a predetermined angle with respect to the first conduit portion. Note that predetermined angle at the bend 830 in one example is about 34 degrees, but can vary from this in other examples, including angles smaller or greater than 34 degrees in one example. The magnitude of the predetermined angle at the bend 830 depends in one example on various factors, including the size of the catheter and/or needle to be inserted into the port conduit, the size of the conduit itself, etc. Note also that the conduit bend 830 serves as a needle-stop feature, preventing the needle 42 from advancing along the conduit 818 past the bend 830.

The second conduit portion 818B of each port body first portion 812A distally extends to a cavity 820A defined by the press-fit junction of the port body first portion and the second portion 812B, as seen in FIG. 25D. Two third conduit portions 818C are defined by the second portion 812B of the port body 812 and extend from each of the cavities 820A in a partially arcuate fluid path to the distally-disposed fluid outlets 824A of the stem 824. In the present example the conduit 818 is sized so as to enable the catheter 40 (FIG. 14A) to pass therethrough and past the cavity 820A.

As mentioned, the cavities 820A, each defined by the junction of the respective first portion 812A and the second portion 812B of the port body 812, each define a space through which the conduit 818 passes and in which is housed a valve/seal assembly 822. In the present example and as best seen in FIGS. 25C and 25D, the valve/seal assembly 822 includes a sealing element, or seal 832, which defines a central hole 832A through which the catheter 40 (FIGS. 14A, 14D) can pass, and a slit valve 834 including two orthogonally intersecting slits 834A through which the catheter also passes. The seal 832 and slit valve 834 are sandwiched together in one example, with the seal disposed proximal to the slit valve, and secured in place within the correspondingly sized cavity 820A as shown in FIG. 25D.

As mentioned, the slits 834A of the slit valve 834 are orthogonally offset from one another by about 90 degrees in the present example, though other relationships are possible, including the use of two single-slit valves sandwiched together with one another. Note that in the present example the slit valve 834 includes a central depression (as in previous examples, such as is shown in FIG. 13A, for instance) to ease the transition of passage of the catheter 40 from the seal 832 to the valve. More than one seal and/or slit valve may be employed in the valve/seal assembly in other examples.

As with previous examples, the seal 832 and slit valve 834 of the valve/seal assembly 822 cooperate to enable fluid-tight passage therethrough of the catheter 40 (*see, e.g.,* FIG. 14A) while also preventing backflow of fluid through the valve/seal assembly. Indeed, in one example the seals disclosed herein prevent fluid flow around the external portion of the catheter when the catheter is disposed through the seal 832, while the valve 834 is suitable for preventing fluid flow when no catheter passes through them. As such, when the catheter 40 is not inserted therethrough the valve/seal assembly 822 seals to prevent passage of air or fluid through the conduit 818. In the present example, the seal 832 and valve 834 are composed of silicone, such as SILASTIC^{®} Q7-4850 liquid silicone rubber available from Dow Corning Corporation, though other suitably compliant materials can be employed. In one example, silicone oil, such as NuSil Technology Med 400 silicone oil, is included with the seal 832 and valve 834 to enhance lubricity and extend component life. In another example, the silicone oil is infused into the silicone.

The port 810 in the present example includes an overmolded portion 836 that covers a portion of the port body 812, including a majority portion of each of the two first portions 818A. The overmolded portion 836 includes silicone, such as SILASTIC^{®} Q7-4850 liquid silicone rubber or other suitably compliant material and surrounds the portions of the body 812 as shown in figs 25A and 25B so as to provide a relatively soft surface for the port 810 and reduce patient discomfort after port implantation within the patient body. The overmolded portion 836 further enables a clinician to suture through one or more of various portions of the overmolded portion to enable the port 810 to be secured within a subcutaneous patient tissue pocket. The overmolded portion 836 further defines a relatively flat bottom surface 836A so as to provide a stable surface for the port 810 in its position within the tissue pocket after implantation into the patient body.

FIG. 25B shows that the first body portions 812A each define a securement ridge 837 that serves as an anchor to prevent relative movement between the overmolded portion 836 and the body 812. The securement ridge 837 can vary in shape, number, configuration, etc. Note that the overmolded portion 836 in one example is molded in a molding process over the body 812. In another example, the overmolded portion 836 is separately formed then adhesively attached to the body 812, such as via Med A adhesive. These and other configurations are therefore contemplated.

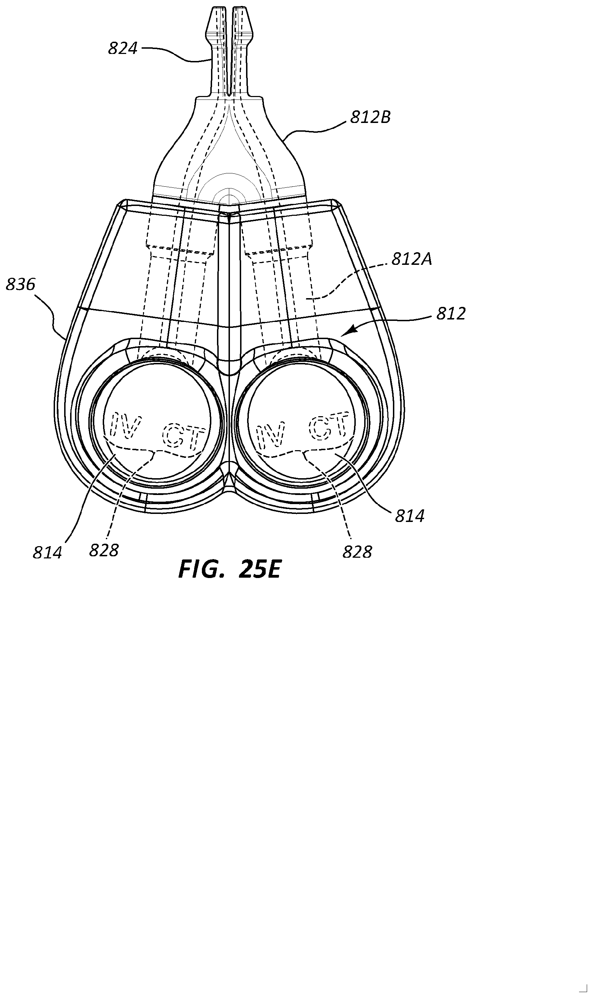

FIG. 25E shows that underside surfaces of the receiving cups 814 include a radiopaque indicia 828 configured to enable the port 810 to be radiographically identified after implantation into the patient body. In the present example each of the indicia 828 includes the letters "IV" and "CT" to indicate suitability of the port 810 to receive peripheral IV catheters and that the port is capable of power injection of fluids therethrough. Of course, a variety of other indicia, including letters, numbers, symbols, etc., may be used.

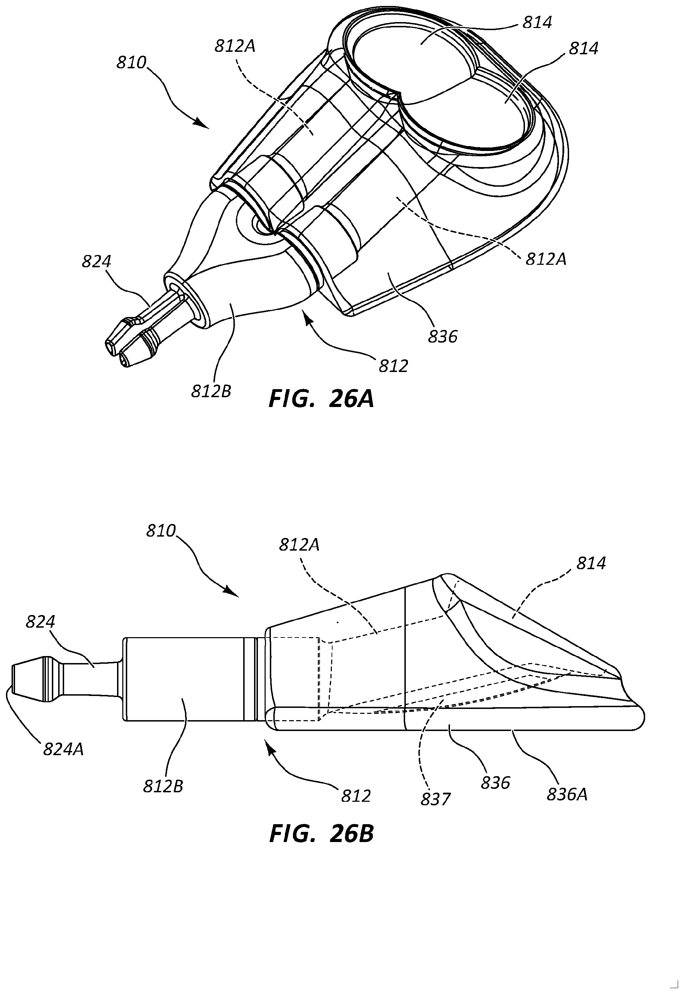

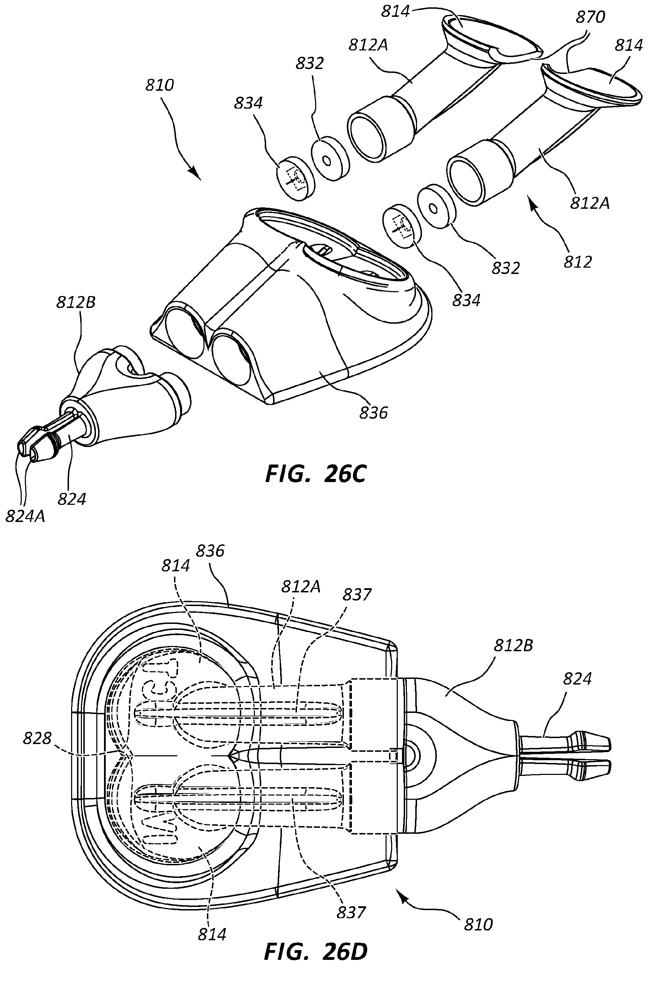

FIGS. 26A-26D depict various details of the port 810 according to another example useful for understanding the invention, wherein the port body 812 defines a relatively slimmer profile than the example shown in FIGS. 25A-25E, made possible by defining a cutout 870 on both receiving cups 814 of each first portion 812A of the port body 812. This enables the receiving cups 814 to reside relatively close to one another. The receiving cups 814 can be joined to one another along the cutouts 870 via welding, adhesive, forming the welding cups together as a single component, etc.

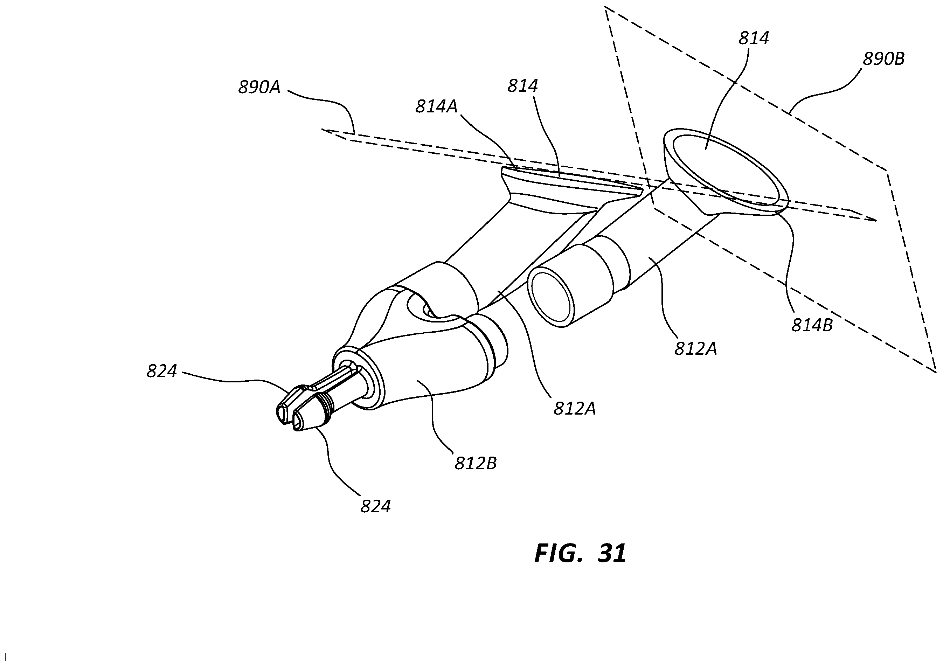

In one example, it is appreciated that the receiving cups 814 can be oriented in other configurations. FIG. 31 gives an example of this, wherein a partially exploded view of the port 810 is shown without the overmolded portion 836 present, and thus including the two first portions 812A and the second portion 812B. As shown, the receiving cups 814 are angled with respect to one another such that a perimeter 814A of a corresponding one of the receiving cups lies in an imaginary plane 890A that is non-parallel to another plane 890B in which a perimeter 814B of the other receiving cup lies. This is in contrast to another example, such as that shown in FIG. 25A, wherein the receiving cups 814 substantially lie in a single imaginary plane. The configuration of FIG. 31 results in the receiving cups 814 being angled away from one another, as shown in FIG. 31 (note that the first body portion 812A shown disconnected (for clarity) from the second body portion 812B is to be connected to the second body portion in substantially the same orientation as shown in FIG. 31). This, in turn, desirably results in a slightly lower height profile for the access port 810, and can also result in the needle 42 inserted therein residing relatively closer to the patient skin, in one example. Note that the receiving cups can be angled in various different configurations in addition to what is shown and described herein.

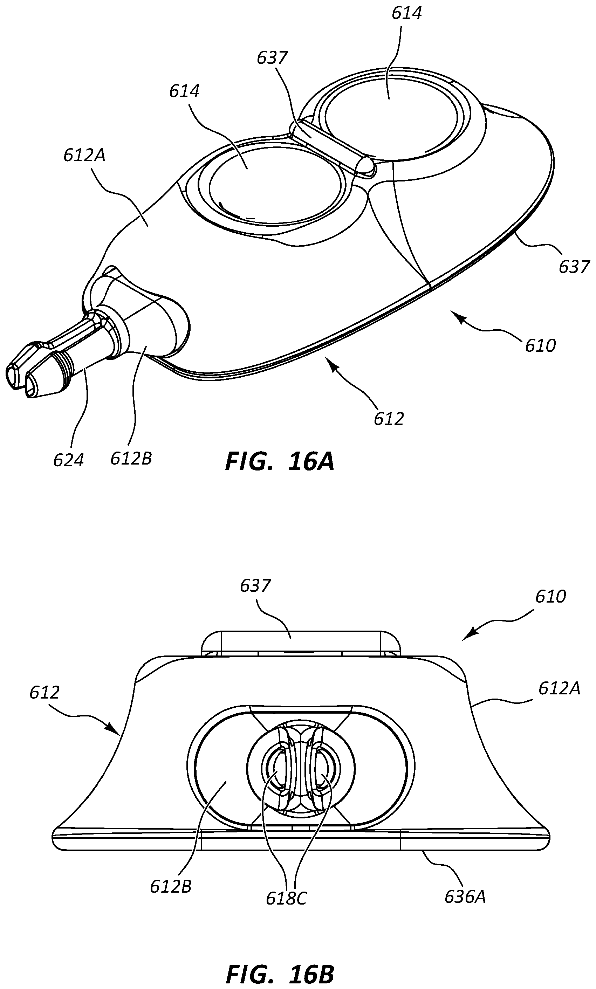

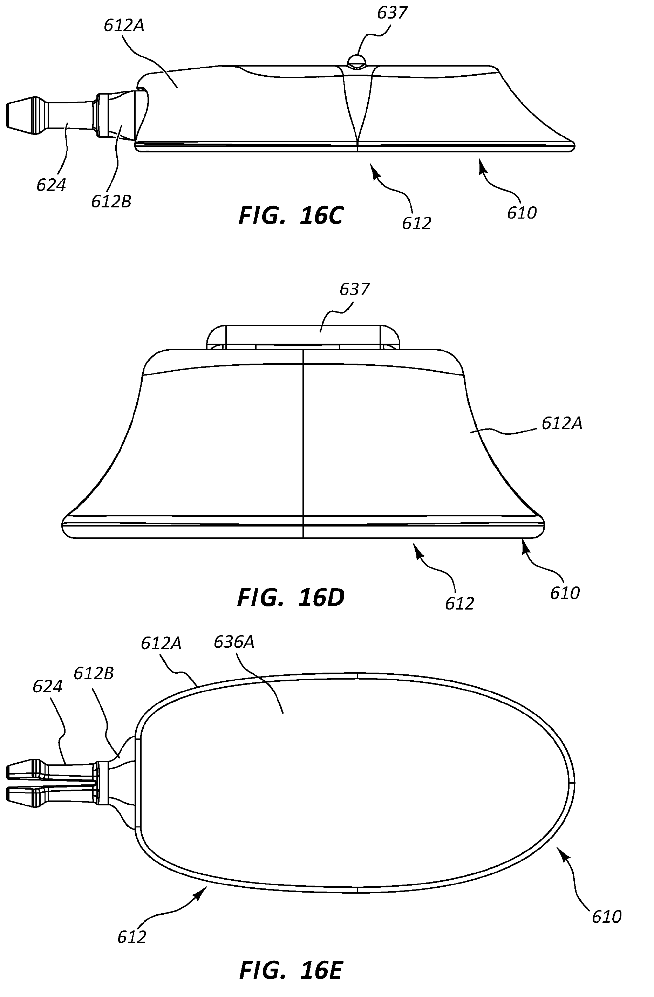

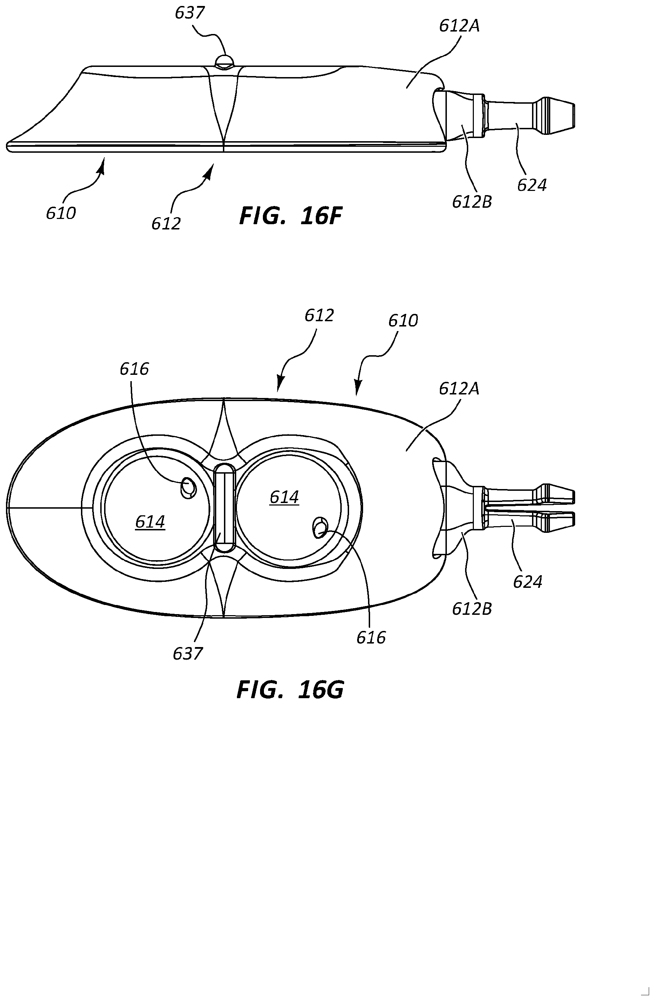

Reference is now made to FIGS. 16A-21B, which depict details of a dual-lumen vascular access device, generally designated at 610, in accordance with one example useful for understanding the invention. As shown, the port 610 includes a body 612 that is defined in the present example by a first portion 612A and a relatively smaller second portion 612B that is partially received within the first portion. In the present example the port body first and second portions 612A, 612B include a metal such as titanium, and as such, the second portion is press fit into engagement with the first portion to define the body, though it is appreciated that the port body can include a variety of other materials, including metals, thermoplastics, ceramics, etc., and can include other joining methods including adhesive, ultrasonic or other welding, interference fit, etc.

The port body first portion 612A defines in the present example two substantially funnel-shaped receiving cups 614 for receiving and directing the catheter-bearing needle 42 (FIG. 14A) to operably connect with the port 610 in a manner similar to that already described above. The receiving cups 614 in the present example are disposed so as to be substantially aligned along a longitudinal axis of the port 610, though other positional arrangements for the receiving cups are possible, including side-by-side, spaced-apart, staggered, etc.

In particular, the substantially funneled-shape of each receiving cup 614 is configured to direct the catheter-bearing needle 42 impinging thereon toward an inlet port 616 that serves as an opening for a respective one of two conduits 618 defined by the port body 612, one conduit for each receiving cup. The open and shallow nature of each receiving cup 614, angled toward the skin surface of the patient enables the receiving cup to present a large, easily accessible target for the needle when introduced into the skin and directed toward the subcutaneously implanted access port 610. FIGS. 16C and 16F further show that the access port 610 defines a relatively low profile height, which enables relatively shorter needle lengths to be used for accessing the subcutaneous access port after implantation.

The port body 612 further defines a palpation feature 637, here configured as a raised surface interposed between the longitudinally aligned receiving cups 614. As mentioned above, the palpation feature 637 is included with the port body 612 to assist a clinician to locate and/or identify the port 610 via finger palpation after implantation under the skin of the patient. Note that a variety of sizes, configurations, numbers, etc., of palpation features can be included on the port. In another example, a guide groove can be defined on each receiving cup 614 to be longitudinally aligned with the inlet port 616 of the conduit 618, as in previous examples.

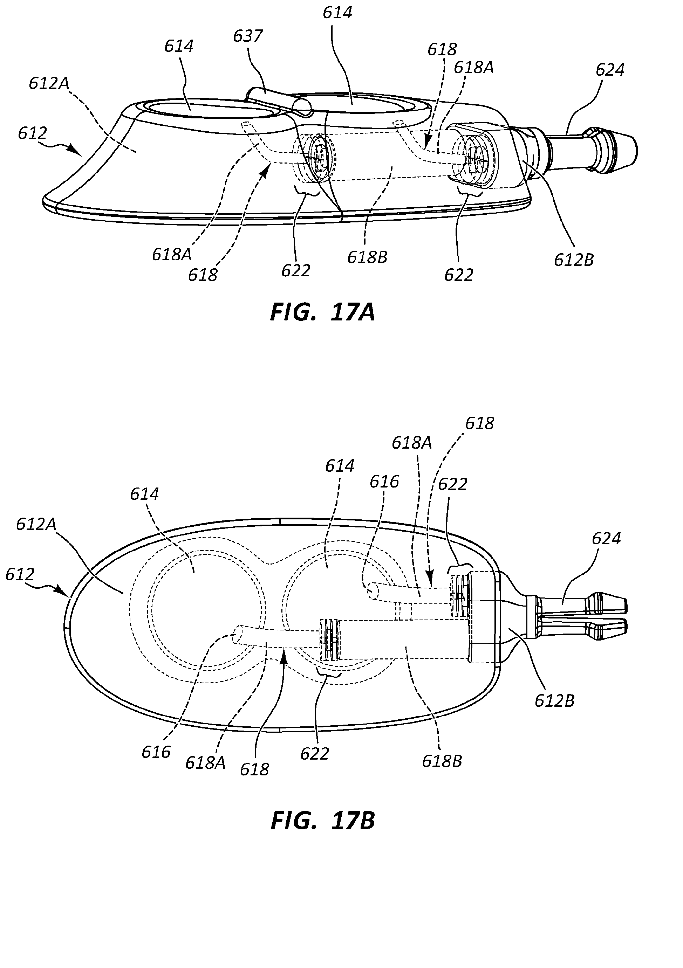

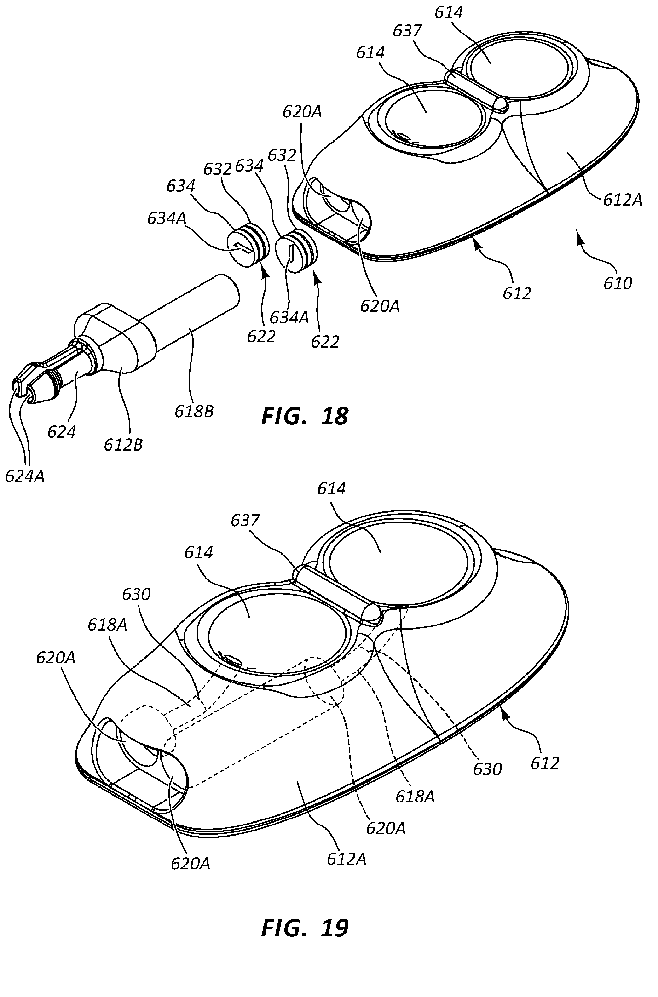

As best seen in FIGS. 17A, 17B, and 19, the port body 612 further defines the above-mentioned two conduits 618, each conduit serving as a pathway into which a transcutaneously inserted catheter can be partially inserted so as to place the catheter in fluid communication both with the port 610 and an indwelling dual-lumen catheter operably attached to two fluid outlets 624A of a stem 624 of the port. As shown, the two conduits 618 of the port body first portion 612A are in fluid communication with their respective receiving cup 614 via the corresponding inlet port 616. A first conduit portion 618A of each conduit 618 distally extends from the respective inlet port 616 in an angled downward direction from the perspective shown in FIG. 17A to a conduit bend 630 (FIG. 19), where the first conduit portion extends distally at a predetermined angle with respect to the first conduit portion proximal to the conduit bend. The magnitude of the predetermined angle at the bend 630 depends in one example on various factors, including the size of the catheter and/or needle to be inserted into the port conduit, the size of the port and the conduit itself, etc. Note also that the conduit bend 630 serves as a needle-stop feature, preventing the needle 42 from advancing along the conduit 618 past the bend 630.

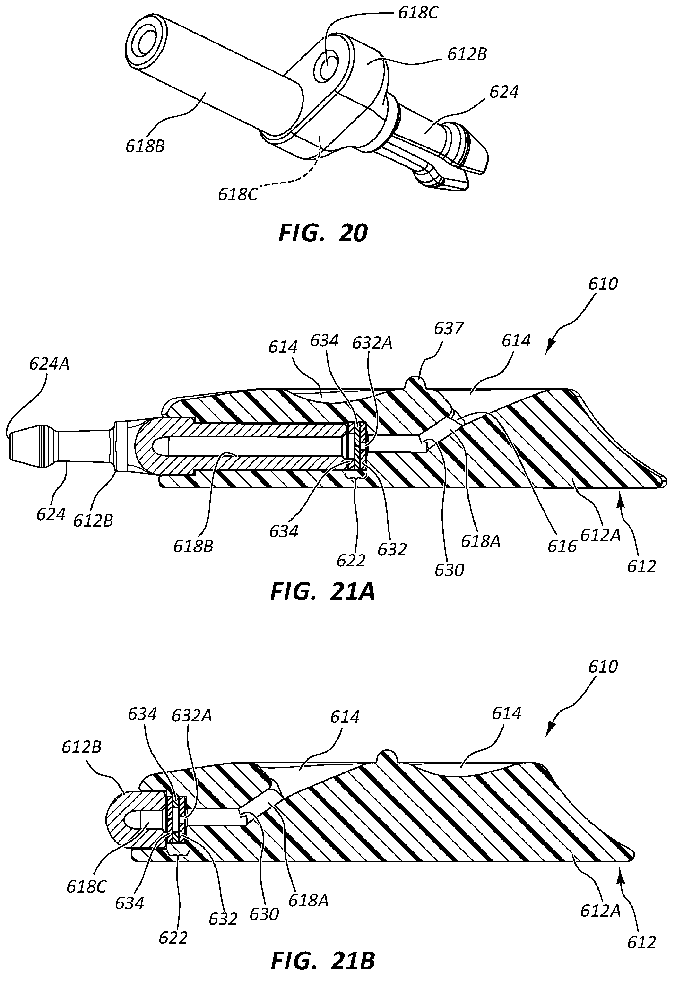

The first portion 618A of the relatively more distal of the two receiving cups 614 extends to a cavity 620A defined by and proximate to the distal portion of the first portion 612A of the port body 612, as best seen in FIGS. 18 and 19. The first portion 618A of the relatively more proximal of the two receiving cups 614 also extends to a cavity 620A that is defined by, but relatively more proximally distant from, the distal portion of the first portion 612A of the port body 612 (FIGS. 18 and 19). A second conduit portion 618B is defined for this latter conduit 618 by the second portion 612A of the port body 612, as seen in FIGS. 17A and 17B and extends distally from its respective cavity 620A until joining with a third conduit portion 618C defined by the second portion 612A of the port body, which extends through the second portion and the stem 624 until terminating at a respective one of the fluid outlets 624A (FIG. 20).

The conduit 618 for the relatively more distal receiving cup 614 extends from the cavity 620A to a third conduit portion 618C defined by the second portion 612A of the port body 612, as seen in FIG. 20, which extends through the second portion and the stem 624 until terminating at a respective one of the fluid outlets 624A. In this way, fluid pathways are defined for each receiving cup 614 from the inlet port 616 to the stem fluid outlet 624A, as depicted in FIGS. 21A and 21B. In the present example the conduit 618 is sized so as to enable the catheter 40 (FIG. 14A) to pass therethrough past the cavity 620A.

As mentioned, the cavities 620A, each disposed in the fluid pathway defined by the various portions of the conduits 618, each define a space through which the conduit 618 passes and in which is housed a valve/seal assembly 622. In the present example and as best seen in FIGS. 17A-18, each valve/seal assembly 622 includes a sealing element, or seal 632, which defines a central hole 632A (FIG. 21B) through which the catheter 40 (FIGS. 14A, 14D) can pass, and two adjacently placed slit valves 634, each slit valve including a single slit 634A (with the valves being arranged such that the slits are orthogonal to one another), through which the catheter also passes. The seal 632 and slit valves 634 are sandwiched together in one example, with the seal disposed proximal to the slit valve, and secured in place within the correspondingly sized cavity 620A as shown in FIGS. 17A and 17B. In another example, the valve/seal assembly includes a single seal and a single, dual-slit valve, as in previous examples.

In the present example, the seal 632 and valves 634 are composed of silicone, such as SILASTIC^{®} Q7-4850 liquid silicone rubber available from Dow Corning Corporation, though other suitably compliant materials can be employed. In one example, silicone oil, such as NuSil Technology Med 400 silicone oil, is included with the seal 632 and valves 634 to enhance lubricity and extend component life. In another example, the silicone oil is infused into the silicone. Also, and as has been mentioned with other examples, other seal/valve configurations can also be employed in the port 610.

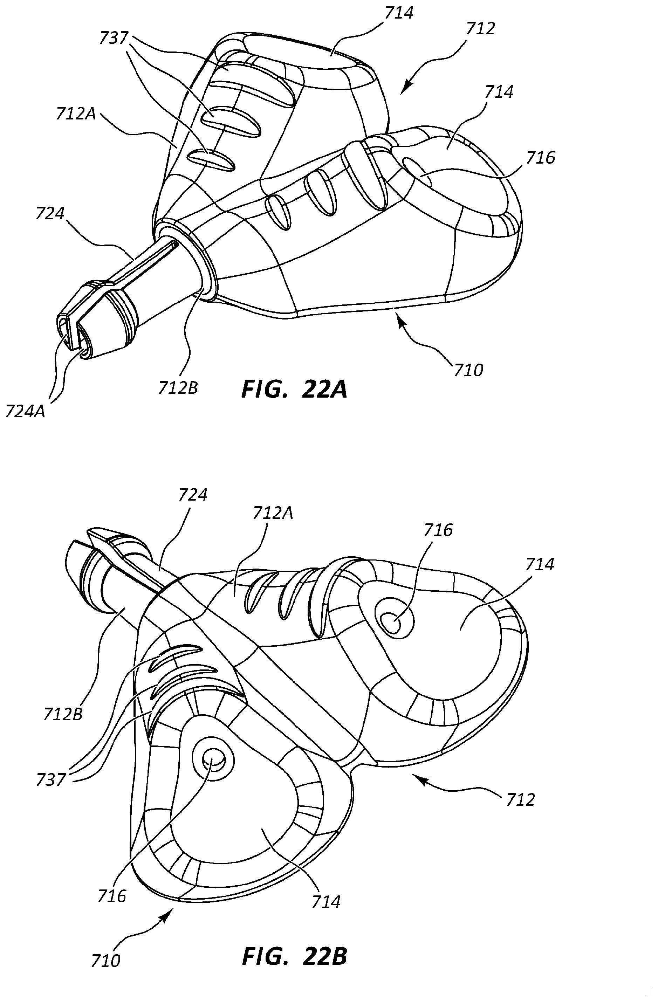

Reference is now made to FIGS. 22A-24, which show various details of a dual-lumen vascular access device, generally designated at 710, in accordance with one example useful for understanding the invention. As shown, the port 710 includes a body 712 that is defined in the present example by a first portion 712A defining the majority of the external portion of the port body and a second portion 712B that is matable to the first portion. In the present example the port body first and second portions 712A, 712B include a metal such as titanium, and as such, the second portion is press fit into engagement with the first portion to define the body 212, though it is appreciated that the port body can include a variety of other materials, including metals, thermoplastics, ceramics, etc., and can include other joining methods including adhesive, ultrasonic or other welding, interference fit, etc.

The port body first portion 712A defines in the present example two substantially concavely-shaped receiving cups 714, side-by-side in a spaced-apart arrangement, for receiving and directing the catheter-bearing needle 42 (FIG. 14A) to operably connect with the port 710 in a manner similar to that already described above. In particular, the substantially concave shape of each receiving cup 714 is configured to direct the catheter-bearing needle 42 impinging thereon toward an inlet port 716 that serves as an opening for a respective conduit 718 defined by the port body 712.

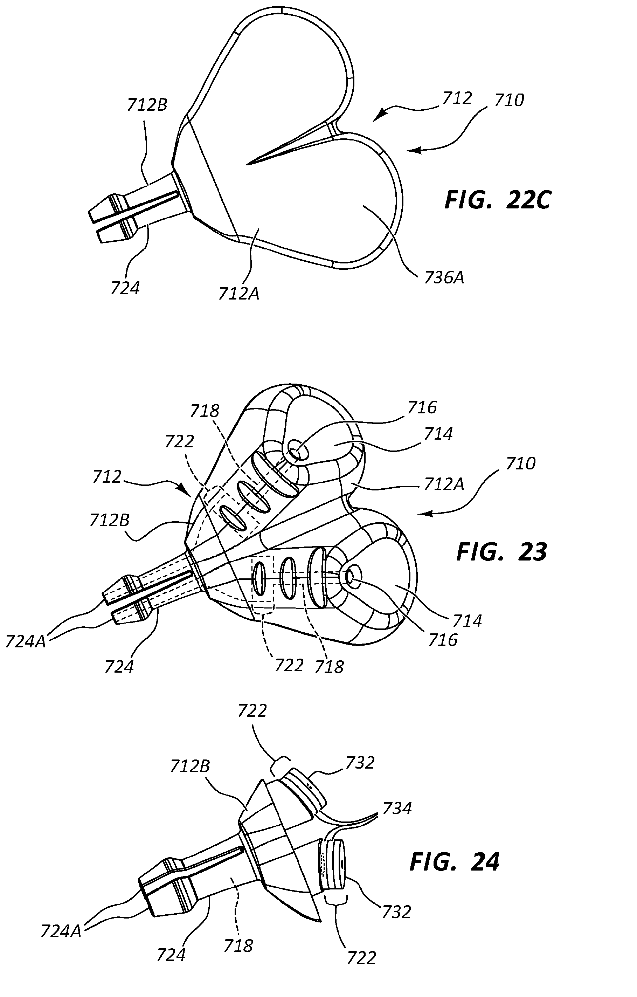

The open and shallow nature of each receiving cup 714, angled toward the skin surface of the patient enables the receiving cup to present a large, easily accessible target for the needle when introduced into the skin and directed toward the subcutaneously implanted access port 710. FIGS. 22A and 22B further show that the access port 710 defines a relatively low profile height, which enables relatively shorter needle lengths to be used for accessing the subcutaneous access port after implantation. FIG. 22C depicts details of a bottom portion of the port body 712. Note that in this and other examples, the receiving cups can define different surfaces, including funnel-shaped, concave-shaped, hemispherical, etc.

The port body 712 includes a plurality of palpation features 737, here implemented as ridges extending distally from the receiving cups 714, to assist a clinician to locate and/or identify the port 710 via finger palpation after implantation under the skin of the patient. Note that a variety of sizes, configurations, numbers, etc., of palpation features can be included on the port.

As best seen in FIGS. 23 and 24, the port body 712 further defines the two conduits 718, each conduit serving as a pathway into which a transcutaneously inserted catheter can be partially inserted so as to place the catheter in fluid communication both with the port 710 and an indwelling dual-lumen catheter operably attached to two fluid outlets 724A of a stem 724 of the port. As shown, each of the two conduits 718 of the port body first portion 712A is in fluid communication with its respective receiving cup 714 via the inlet port 716 and extends distally to a valve/seal assembly 722 disposed in a cavity cooperatively defined by the junction of the port body first portion 712A and the second portion 712B. As with other examples herein, each conduit 718 distally extends from the respective inlet port 716 in an angled downward direction from the perspective shown in FIG. 23 to a conduit bend before continuing to the cavity wherein is disposed the valve/seal assembly. Note that the conduit bend can desirably serve as a needle-stop feature, preventing the needle 42 from advancing along the conduit 718 past the bend. The conduits distally extend past the valve/seal assembly 722 and through the port body second portion 712B to the fluid outlets of the stem 724. In the present example the conduit 718 is sized so as to enable the catheter 40 (FIG. 14A) to pass therethrough past the valve/seal assembly 722.

As mentioned, the cavities, each defined by the junction of the respective first portion 712A and the second portion 712B of the port body 712, each define a space through which the conduit 718 passes and in which is housed the valve/seal assembly 722. In the present example and as best seen in FIGS. 23 and 24, each of the two valve/seal assemblies 722 includes a sealing element, or seal 732, which defines a central hole through which the catheter 40 (FIGS. 14A, 14D) can pass, and two slit valves 734, each including a single slit and positioned adjacent each other such that the slits are substantially orthogonal to one another, through which the catheter also passes. The seal 732 and the slit valves 734 are sandwiched together in one example, with the seal disposed proximal to the slit valves, and secured in place within the correspondingly sized cavity as shown in FIGS. 23 and 24.

As mentioned, the slits of the slit valves 734 are orthogonally offset from one another by about 90 degrees in the present example, though other relationships are possible, including the use of a single slit valve including two orthogonal slits. These and other modifications to this and the other valve/seal assembly examples herein are therefore contemplated.

As with previous examples, the seal 732 and slit valves 734 of the valve/seal assembly 722 cooperate to enable fluid-tight passage therethrough of the catheter 40 (*see, e.g.,* FIG. 14A) while also preventing backflow of fluid through the valve/seal assembly. Indeed, in one example the seals disclosed herein prevent fluid flow around the external portion of the catheter when the catheter is disposed through the seal 732, while the valve 734 is suitable for preventing fluid flow when no catheter passes through them. As such, when the catheter 40 is not inserted therethrough the valve/seal assembly 722 seals to prevent passage of air or fluid through the conduit 718. In the present example, the seal 732 and valve 734 are composed of silicone, such as SILASTI^{C®} Q7-4850 liquid silicone rubber available from Dow Corning Corporation, though other suitably compliant materials can be employed. In one example, silicone oil, such as NuSil Technology Med 400 silicone oil, is included with the seal 732 and valve 734 to enhance lubricity and extend component life. In another example, the silicone oil is infused into the silicone.

Though not explicitly shown here, the port 710, as with other examples herein, can include radiopaque indicia configured to enable the port to be radiographically identified after implantation into the patient body. In one example, the indicia include the letters "IV" and "CT" to indicate suitability of the port 710 to receive peripheral IV catheters and that the port is capable of power injection of fluids therethrough. Of course, a variety of other indicia, including letters, numbers, symbols, etc., may be used.

Though single and dual-port configurations have been described herein, it is appreciated that ports including more than two receiving cups are contemplated. Note also that certain of the receiving cups described herein are described as funnel shaped, while other receiving cups are described herein as concavely shaped. It is noted that that the receiving cups can interchangeably include aspects of one or the other, or both, of these receiving cup shapes, according to a particular example.

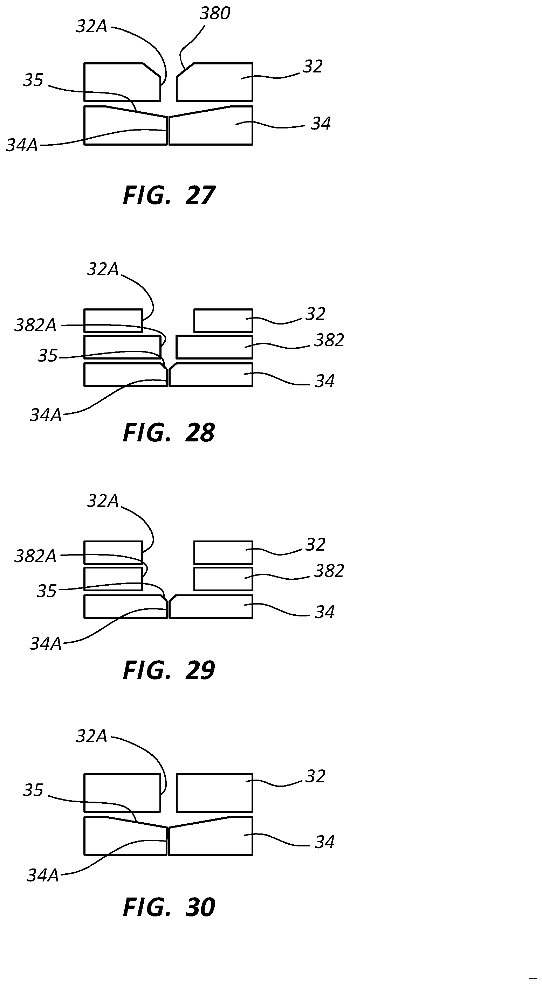

FIGS. 27-30 depict details of various possible configurations for the valve/seal assembly, according to examples useful for understanding the invention. In FIG. 27, the seal 32 includes a central depression 380, similar but relatively steeper than the depression 35 of the valve 34. In FIG. 28, two seals are included - the seal 32 and a second seal 382 interposed between the seal 32 and the valve 34. The second seal 382 includes a central hole 382A that includes a diameter smaller relative to the hole 32A of the seal 32. FIG. 29 includes a similar configuration, but the hole 382A is similar in size to the hole 32A. A small central depression 35 is included on the valve 34 in both FIGS. 28 and FIG. 29.

In FIG. 30, the seal 32 includes a relatively small-diameter central hole 32A, and the valve 34 includes a relatively large central depression 35. Note that the valve/seal assemblies shown in FIGS. 27-30 are oriented in the figures such that the catheter pierces the seals and valves in a direction corresponding from the top of the page toward the bottom of the page.

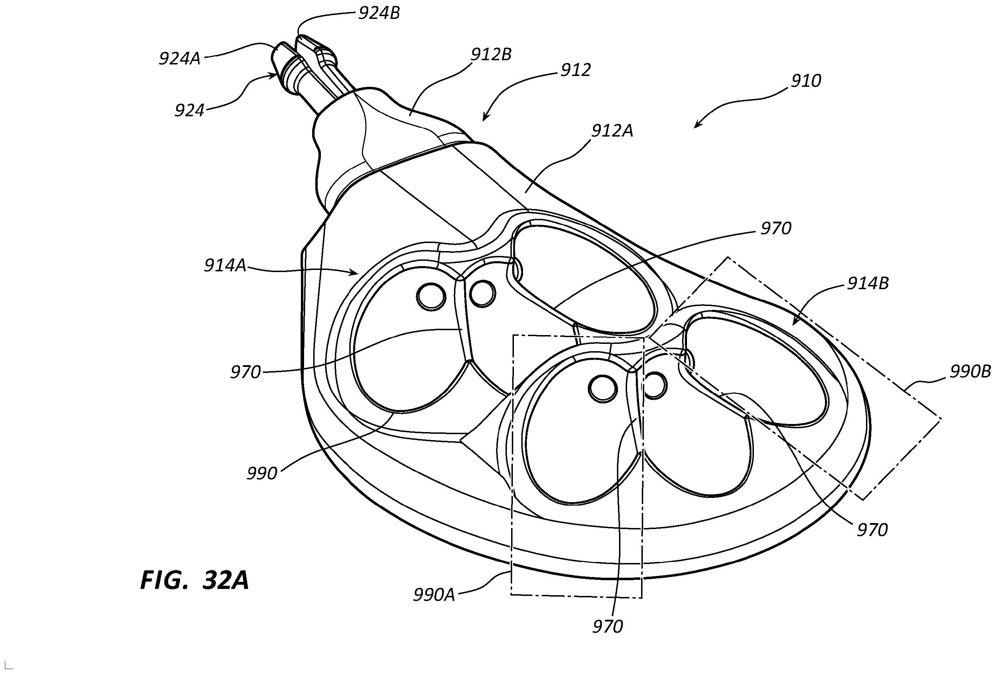

Reference is now made to FIGS. 32A-32B, which show various details of a multi-lumen vascular access device, generally designated at 910 in accordance with one embodiment. As shown, the port 910 includes a body 912 that is defined in the present embodiment by a first portion 912A and a relatively smaller second portion 912B that is partially received within the first portion 912A. In the present embodiment, the port body first and second portions 912A, 912B include a metal such as titanium, and as such, the second portion is press fit into engagement with the first portion to define the body 912. However, it will be appreciated that the port body can include a variety of other suitable materials, including metals, thermoplastics, ceramics, etc., and can include other joining methods including snap-fitted, adhesive, ultrasonic or other welding, interference fit, etc., as discussed herein.

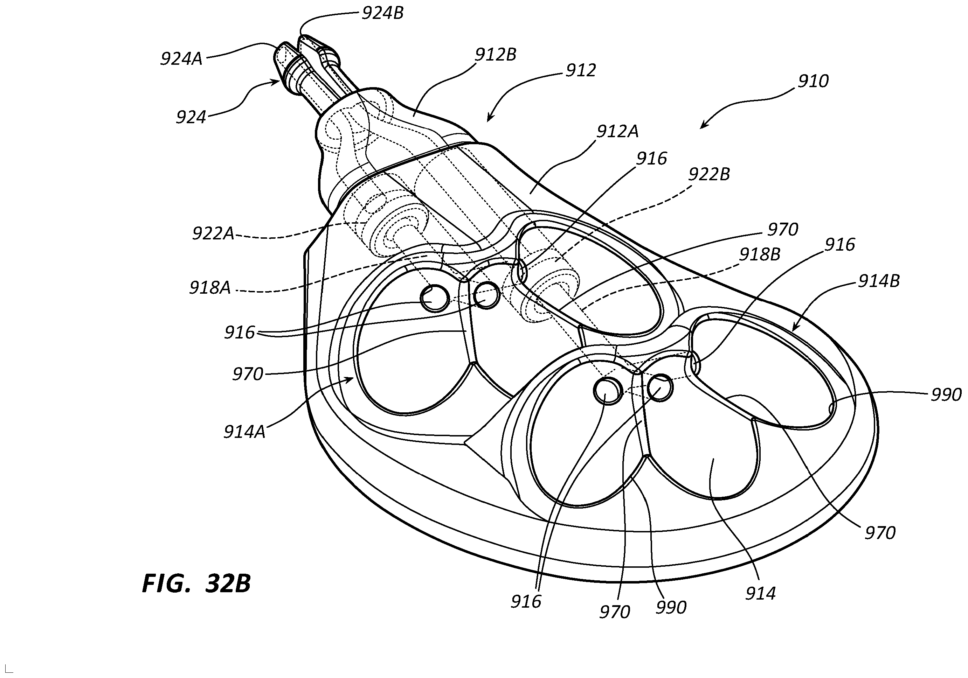

The port body first portion 912A defines in the present embodiment a plurality of substantially funnel-shaped receiving cups 914 for receiving and directing the catheter-bearing needle 42 (FIG. 14A) to operably connect with the port 910 in a manner similar to that already described above. The receiving cups 914 in the present embodiment are disposed in sets, or groups, so as a first set of receiving cups 914A and second set of receiving cups 914B are substantially aligned along a longitudinal axis of the port 910, such that a first set 914A is proximal to second set of receiving cups 914B, though other positional arrangements for the receiving cups are possible, including side-by-side, spaced-apart, staggered, etc. As shown in FIG. 32B, each set of receiving cups 914A, 914B include three individual receiving cups 914, although it will be appreciated that a greater or fewer number of receiving cups 914 within each set 914A, 914B are contemplated and fall within the scope of the present invention.

In an embodiment, port body 912 includes sets of receiving cups 914A, 914B that include individually defined receiving cups 914 similar to those shown in FIG. 25A. In an embodiment, as shown in FIG. 32A, each of the receiving cups 914 within a set 914A, 914B can be joined to one another along cutouts 970. This enables the receiving cups 914 to reside relatively close to one another and provide port body 912 with a relatively slimmer profile than that of an embodiment where receiving cups 914 are individually defined. The receiving cups 914 of each set 914A, 914B can be joined to one another along the cutouts 970 via welding, adhesive, forming the welding cups together as a single component. In an embodiment each set of receiving cups 914A, 914B are formed as a single monolithic piece. In an embodiment, port body second portion 912B is formed as a single monolithic piece.

The substantially funneled-shape of each receiving cup 914 is configured to direct the catheter-bearing needle 42 impinging thereon toward a corresponding inlet port 916 for each cup 914. Each set of receiving cups 914A, 914B then communicates with a single conduit 918, i.e. conduit 918A, 918B respectively. The conduits 918A, 918B, in turn communicate with a corresponding stem fluid outlet 924A, 924B of port stem 924, as described herein. Further, each of the conduits 918 can include valve/seal assemblies 922, also as described herein. Accordingly, a given conduit, e.g. 918A or 918B, can accessed by any of the receiving cups within a corresponding set of receiving cups 914A, 914B. One embodiment of suitable internal inlet port 916 / conduit 918 routing is disclosed in FIG. 32B.

Advantageously, this allows a user to access a conduit 918 via multiple needle entry points. Accordingly, the port 910 is suitable for implantation under the skin of a dialysis patient, or patient undergoing similar extracorporeal treatments that require infusion and removal of fluids from the vasculature. Multiple needle entry points can be used and can be alternately selected over the course of multiple dialysis treatments so that no single locus of the patient's skin needs to be consecutively penetrated by a needle in order to access a given conduit 918.

In an embodiment, each the receiving cups 914 within a set can be oriented along a similar plane, such that they are co-aligned. In an embodiment, each of the receiving cups 914 within a set are angled with respect to one another such that a perimeter 990 of a first receiving cup 914 lies in an imaginary plane 990A that is non-parallel the planes defined by the perimeters of the other receiving cups 914 within the set, for example plane 990B defined by a second receiving cup 914, as described herein (FIG. 31). Such a configuration results in each of the receiving cups 914 within a set 914A, 914B being angled away from one another. This, in turn, desirably results in a slightly lower height profile for the access port 910, and can also result in the needle 42 inserted therein residing relatively closer to the patient skin. Further, the angled receiving cups 914 provide a greater skin surface with which to access the port 910. Accordingly, repeated access can be achieved using a greater number of needle access points so that no single locus of the patient's skin needs to be consecutively penetrated by a needle, allowing previous sites to heal. Note that the receiving cups can be angled in various different configurations in addition to what is shown and described herein.

Although two sets of three receiving cups each are shown, it will be appreciated that any number of receiving cups, or number of sets thereof, fall within the scope of the present invention. Accordingly, in an embodiment, one set of receiving cups may be configured for blood withdrawal, and the other set configured for blood return.

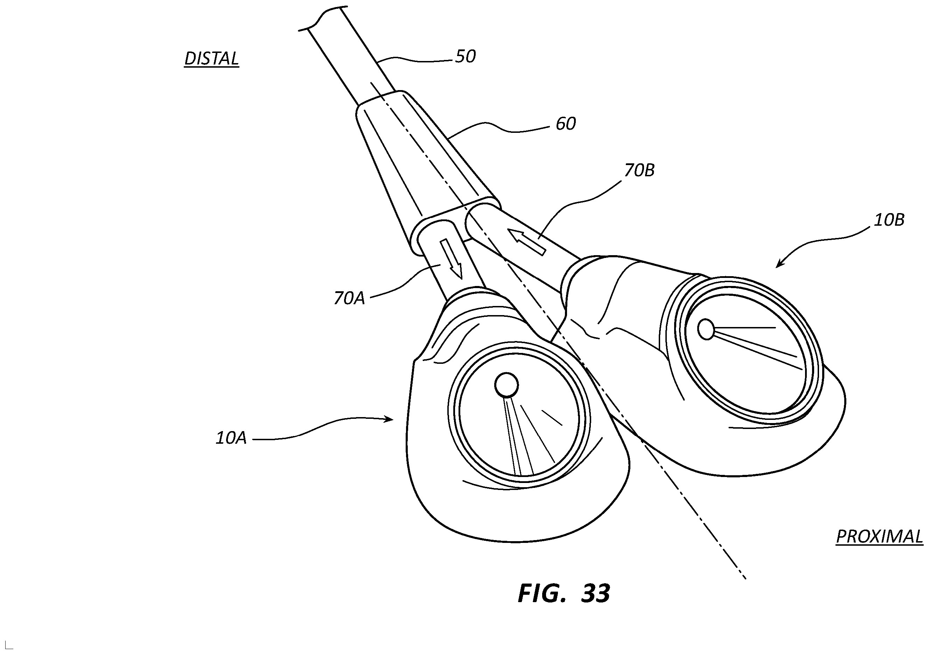

Reference is now made to FIG. 33 which illustrates an embodiment of a subcutaneous catheter assembly. The catheter assembly comprises a catheter 50, a bifurcation hub 60, an extension leg 70, such as extension legs 70A, 70B, and a port 10. The catheter 50 can be a multi-lumen catheter, such as a dual lumen dialysis catheter where each lumen is fluidly connected with an extension leg 70A, 70B. A port 10 is fluidly connected with a proximal end of the extension leg 70 and can be configured for receiving dialysis needles or large gauge over-the-needle intravenous catheters. Accordingly, a first port 10A can be accessed to fluid removal and a second port 10B can be access for fluid return. Each port 10 can include palpation features, indicia, guide grooves, radiopaque markers, or other features of other embodiments as disclosed herein.

Advantageously, the length and flexibility of the extension legs 70 allow an amount of variation in positioning of the ports 10A, 10B relative to each other. Accordingly, the ports can be positioned to alter the access locus on the patient's skin without having to reposition the entire device. Further, individual ports 10 can be replaced as needed without having to replace the entire device. It will be appreciated that alternate embodiments of port as disclosed herein can be used in place of port 10. Further, catheters with different numbers of lumens and gauge sizes can also be used and fall within the scope of the present invention.

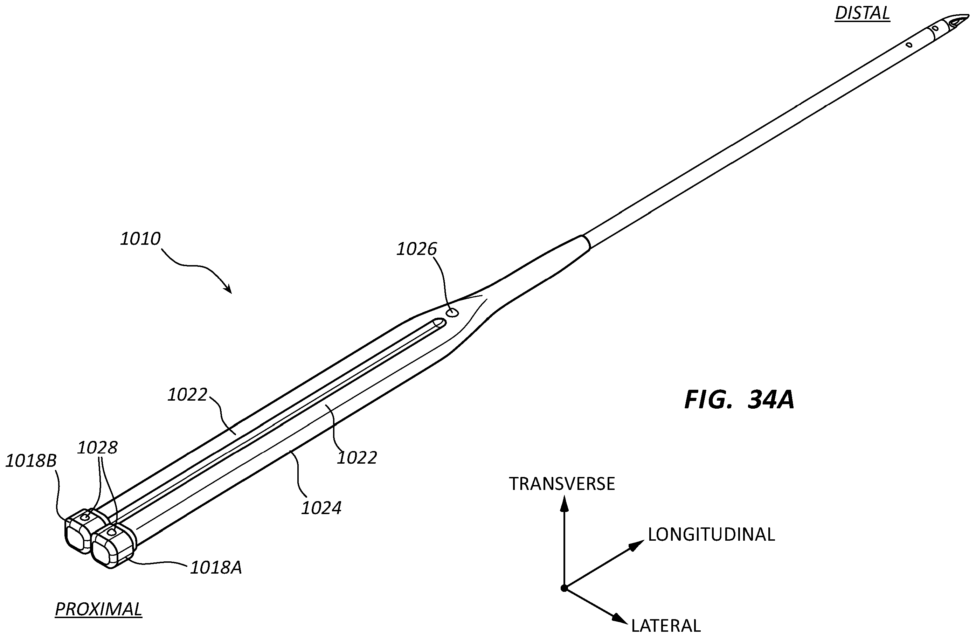

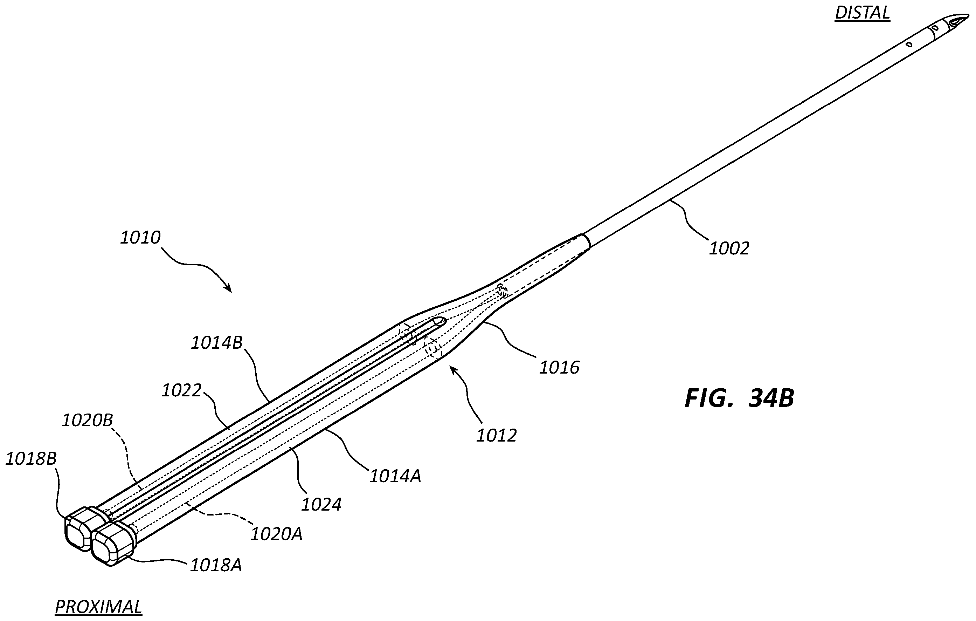

Reference is now made to FIGS. 34A-34B, which show various details of a multi-lumen vascular access device, generally designated at 1010 in accordance with one example useful for understanding the invention. The port 1010 is configured to be surgically implanted under the skin of a patient, and includes a port body 1012 fluidly connected with an in-dwelling, multi-lumen catheter 1002 disposed within the vasculature of a patient. The port body 1012 comprises two elongate, compliant arms, 1014A, 1014B, each of which define a lumen 1020A, 1020B therein, which are fluidly connected with a lumen of the in-dwelling, multi-lumen catheter 1002, by way of a bifurcation hub 1016. The arms 1014, including the lumens 1020 disposed therein, extend proximally from a proximal end of the bifurcation hub 1016 along a longitudinal axis. Although FIGS. 34A-34B show two arms extending side by side along a longitudinal axis, other numbers of arms 1014 and configurations thereof are contemplated. For example, at least one arm 1014 can extend at an angle relative to the longitudinal axis. A proximal end of each of the arms 1014A, 1014B, terminates in an end cap 1018A, 1018B. The end cap 1018 can be formed of the same or of a different material from that of the arms and can be attached thereto using adhesive, welding, bonding, or similar suitable techniques. In an example, the caps are formed monolithically with the arms 1014. The port 1010, or portions thereof, can be formed of any suitable biocompatible material, as discussed herein.

The port 1010, or portions thereof, can include palpation features 1026. For example, bifurcation hub 1016, end caps 1018, or combinations thereof can include palpation features that can indicate a position and/or orientation of the port body 1012, arms 1014, or the like, as discussed herein. Further, port 1010, portions thereof, or indicia included therewith, can include metals, such as titanium, that are radiopaque thus allowing the port 1010 to be located and identified using a suitable imaging modality, as discussed herein. For example, end cap 1018, arm 1014, bifurcation hub 1016, or combinations thereof, can include a radiopaque material to indicate a position and/or orientation of the port 1010, subsequent to subcutaneous implantation, using a suitable imaging modality, e.g. x-ray, CAT, PET, MRI, ultrasound, or the like. To note, the bifurcation hub 1016 and the end cap 1018 can include differently shaped palpation features 1026 / radiopaque indicia to indicate to a user a flow direction. A needle 42 can then be inserted at an obtuse angle relative to the flow direction. It will be appreciated that the needle 42 can also be inserted substantially orthogonal to the longitudinal axis of the port 1010.

A portion of the arms 1014 can include a self-sealing, needle penetrable material, such as silicone, or the like. The self-sealing, needle penetrable material can be disposed in an upper wall 1022 of the arms 1014. Further, a lower wall 1024 of the arms can include a needle-impenetrable material, for example, plastic, metal, or the like. The upper and lower walls 1022, 1024 can be defined relative to the transverse axis. As noted the arms 1014 are compliant, this enables the arms to conform to the specific contours of the patient's body where it is subcutaneously implanted. Accordingly, while the material of the lower wall 1024 is needle impenetrable, the material is also sufficiently compliant to conform to the patient's body. In an example, a portion of the inner surface of the lumen 1020 includes a needle impenetrable material, such as those discussed herein, that prevents the distal end of a needle from gouging the inner surface of the lumen when impinging thereon. This, in turn, prevents the undesirable creation of material flecks dug by the needle.

After locating the port 1010 via through-skin palpation or imaging, a clinician uses the catheter-bearing needle 42 to pierce a skin surface 44 and an upper wall of the port arm 1014, the latter including a needle-penetrable material. The needle 42 is inserted until a distal tip 42A thereof impinges on a lower wall 1024 of the arm 1014, which is formed of a needle-impenetrable material.

The needle 42 can then be proximally backed out a small distance, and the catheter 40 advanced over the needle such that the catheter bends and advances into the lumen 1020 of the arm 1014. Once the distal end 40A of the catheter 40 is in fluid communication with the arm lumen 1020, further advancement can cease and fluid transfer through the catheter 40 and port 1010 can commence, including infusion and/or aspiration through the stem 24. Once fluid transfer is completed, the catheter 40 can be withdrawn proximally and then withdrawn through the surface 44 of the skin and out of the patient.

Advantageously, the port 1010 provides a relatively large area with which a clinician can access the port while maintaining a low profile. This allows a clinician to access the dialysis device at different positions during the course of multiple dialysis treatments, by inserting the needle in different locations along the arms 1014.

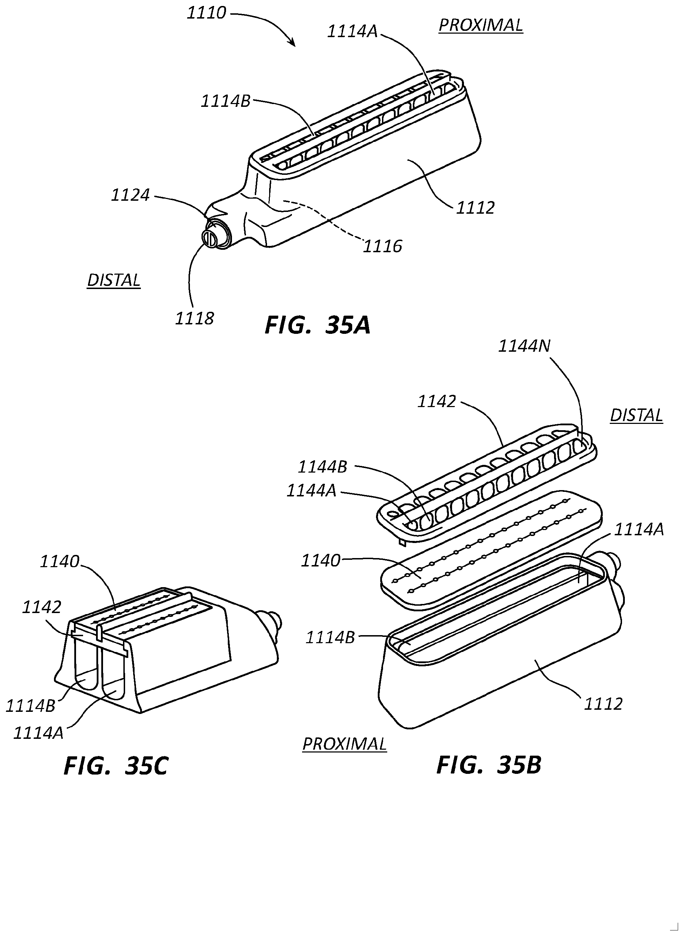

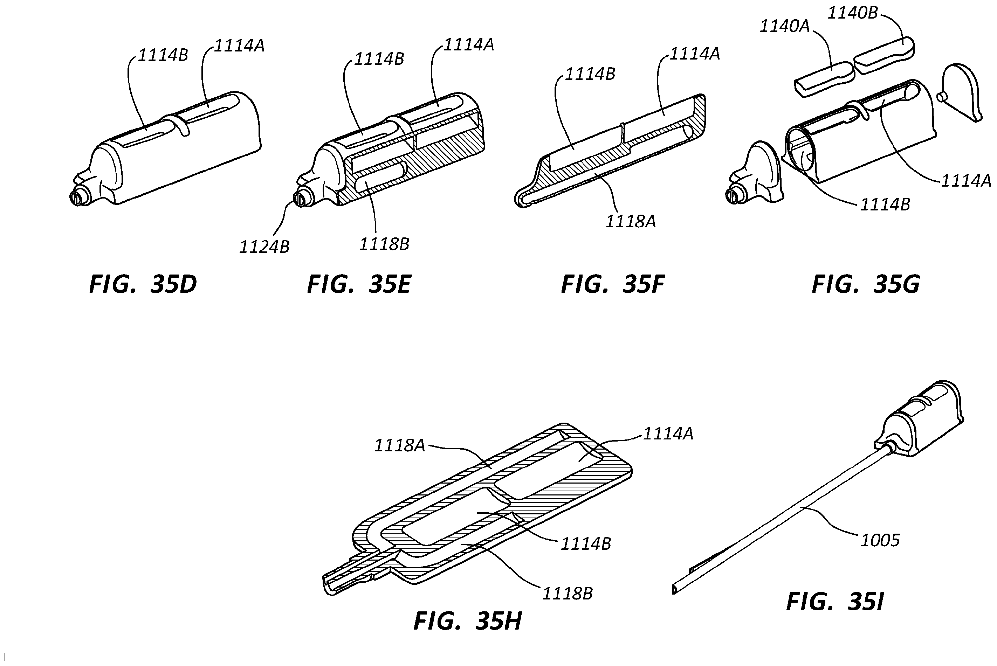

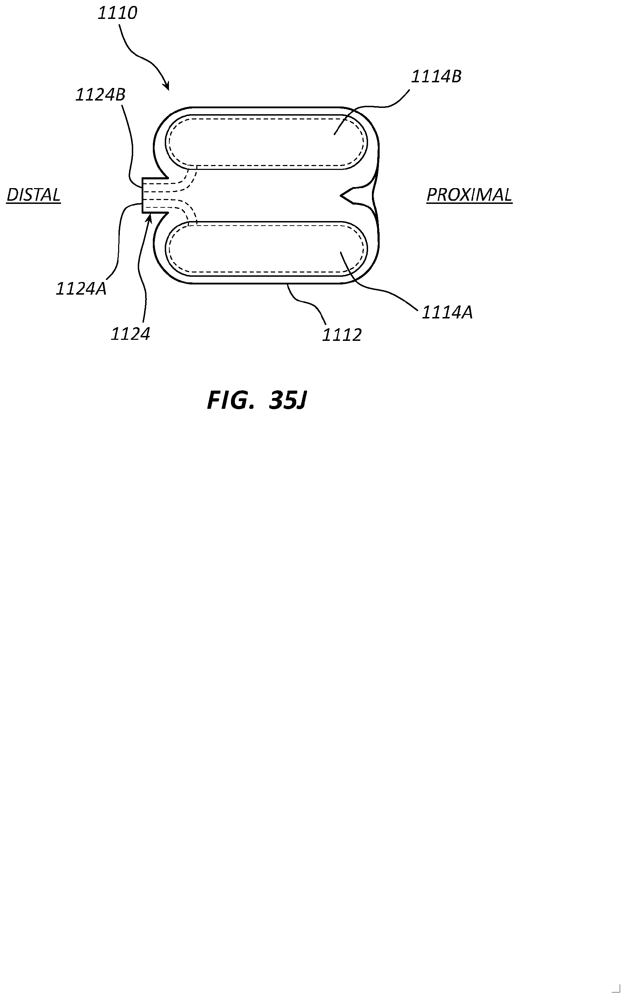

Reference is now made to FIGS. 35A-35J, which show various details of a vascular access dialysis device, generally designated at 1110, in accordance with one embodiment. The port 1110 is configured to be surgically implanted under the skin of a patient, and includes a port body 1112 fluidly connected at a distal end with an in-dwelling, multi-lumen catheter 1002 disposed within the vasculature of a patient. The port body 1112 defines an elongate chamber 1114, such as a first and second elongate chamber 1114A, 1114B. Each chamber is in fluid communication with a lumen of the in-dwelling catheter 1002 by way of conduit 1118, defined in port body 1112, which extends from chamber 1114 to a fluid outlet of stem 1124.

Each elongate chamber 1114 can extend longitudinally in a side by side arrangement. In an embodiment, as shown in FIG. 35F, each elongate chamber 1114 can be arranged in tandem such that one is more proximal than the other, as will be discussed in more detail herein. A lower surface of each chamber 1114 can be shaped as an elongate funnel shape so as to direct a needle impinging thereon towards an inlet 1116 of conduit 1118. In an embodiment, the chamber defines a substantially flat or even lower surface extending along the longitudinal axis. In an embodiment, the chamber defines a U-shaped cross sectional shape as shown in FIG. 35C.

Each chamber 1114 includes a septum 1140, formed of a self-sealing, needle-penetrating material, such as silicone. The port 1110 includes a needle guide 1142 disposed either above or below the septum 1140. The needle guide 1142 can be formed of a needle impenetrable material. In an embodiment, the needle guide 1142 can be formed either as a separate piece from that of port body 1112 or formed monolithically therewith. In an embodiment, the needle guide 1142 be formed as a separate piece from that of the septum 1140 and disposed either above or below the septum 1140. In an embodiment, the septum 1140 is overmolded onto the needle guide 1142 such that the needle guide is disposed within the septum 1140. In an embodiment, the needle guide 1142 includes a rail that longitudinally bisects the septum and laterally divide the septum into a plurality of distinct access areas, or openings.

The needle guide 1142 can guide the clinician to penetrate the septum at different positions, thereby avoiding repeated needle penetrations being concentrated at a single locus. The elongate wells 1114 and associated septa, provide a larger area with which to access the port while also maintaining a slim overall profile. The needle guide 1142 can guide a clinician to access the port at a different position, thus promoting tissue healing. For example, dialysis is performed every 2-3 days, the clinician can access the device at a first position 1144A proximate the proximal end of the needle guide 1142. During subsequent dialysis treatments, the clinician can use the needle guide 1142 to direct subsequent access points, or openings, at increasingly distal positions from the first 1144A, such as position 1144B. Accordingly, subsequent access points can migrate distally until the most distal positon is reached 1144N. At which point the skin adjacent a first access point 1144A will have had a chance to heal and the clinician can re-access the initial access point 1144A. Further, the width of the wells 1114 and associated septa 1140 can allow some variation in needle access within a given position 1144 so that the septum is not traversed in exactly the same position each time, thus improving septum longevity.

In an embodiment, as shown in FIGS. 35D-I, each elongate chamber 1114 can be arranged in tandem such that a first chamber 1114A is more proximal than a second chamber 1114B. In such an example, the proximal most chamber 1114A can include a conduit 1118A, defined by the port body 1112, which extends past the more distal chamber 1114B and is fluidly connected with the stem 1124. FIG. 35F shows a first vertical cut away view of the port 1110 where a first chamber 1114A includes a first conduit 1118A extending through first side of the port 1110 and connecting with a first fluid outlet 1124A at the stem 1124. FIG. 35E shows a second vertical cut away view of the port 1110 where a second chamber 1114B includes a second conduit 1118B extending through second side of the port 1110 and connecting with a second fluid outlet 1124B at the stem 1124. In an exemplary embodiment, FIG. 35H shows a horizontal cutaway view of an internal chamber 1114 / conduit 1118 routing. Advantageously, the tandem configuration allows for a wider septa 1140, providing more variation in injection sites at a given position. As such, a particular injection locus on a septum is not degraded from repeated needle penetrations, thereby promoting septa longevity.

It will be appreciated that the port body 1112 can be formed of a suitable biocompatible material, as discussed herein. The port body 1112 can be formed of a needle impenetrable material, optionally each chamber 1114 can include a needle impenetrable material lining an inner surface thereof, as discussed herein. As shown, port 1110 includes two wells 1114 formed in a port body 1112 as a single monolithic piece, although it will be appreciate that any number of wells can be formed in the port 1110 and fall within the scope of the present invention. In an embodiment, the port 1110 can include a single chamber 1114 formed in the port body 1110. In an embodiment, the port 1110 can include wells 1114 formed as separate structures that are each connect to a lumen of a multi-lumen catheter, or an extension leg of a bifurcated catheter. In an embodiment, each chamber can be designed with different characteristics for different purposes. For example, a first chamber can be designed for blood withdrawal and a second chamber for blood return, or they may be reversibly separable. As in other embodiments, one side may be used for blood withdrawal and the other side for blood return.

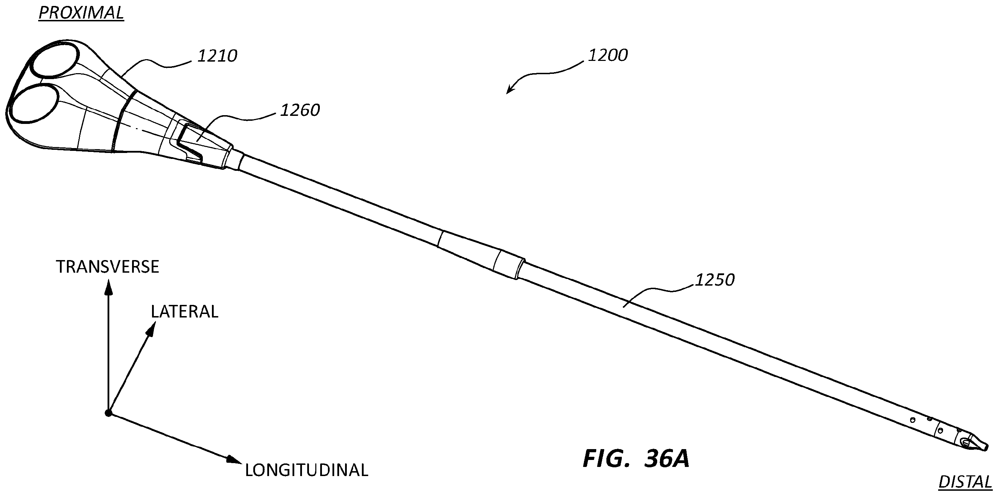

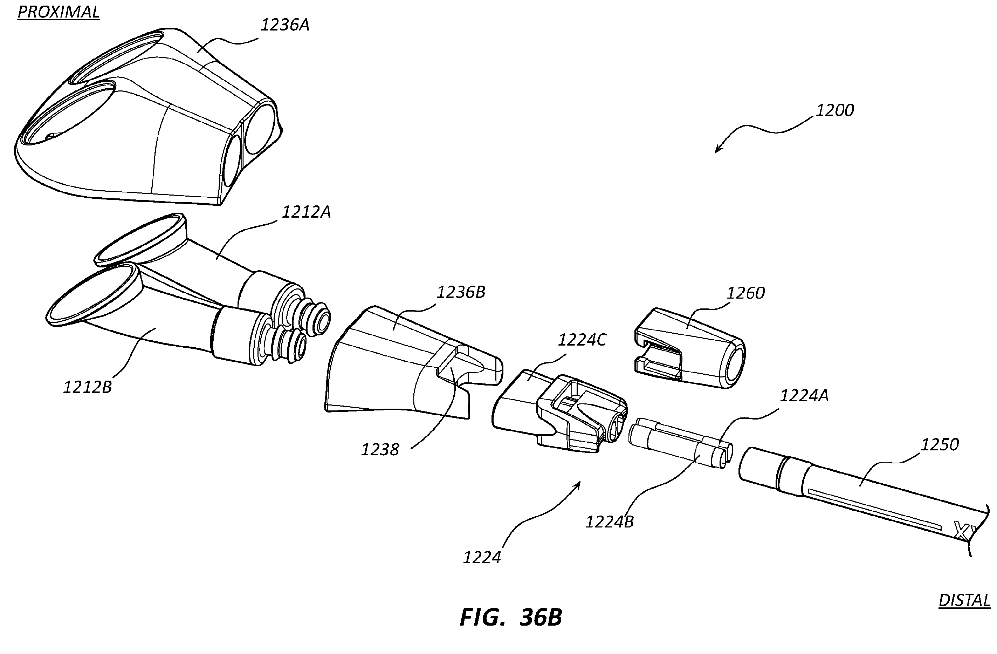

Reference is now made to FIG. 36A-37E, which shows details of an indwelling catheter assembly 1200, in accordance with one example useful for understanding the invention. The catheter assembly 1200 includes a port 1210 fluidly connected to a catheter 1250 by way of locking member 1260. FIG. 36B shows an exploded view of the catheter assembly 1200 including the port 1210, the locking member 1260 and a proximal end of the catheter 1250. The port 1210 includes a body 1212 that is defined by a similarly shaped first conduit 1212A and second conduit 1212B. A distal end of each of the first and second conduits 1212A, 1212B engages a distal portion 1236B of an outer shell 1236. The distal end of the outer shell 1236 includes a distal receiving slot 1238 which engages a proximal end of a stem assembly 1224. The stem assembly 1224 includes a housing 1224C which is configured to receive a first and second stem 1224A, B at a distal end thereof.

Each of the first and second conduits 1212A, B, outer shell portions 1236A, B, and stem assembly 1224, can be press fitted into engagement with each other. Further, the first and second conduits 1212A, 1212B, can include a metal, such as titanium. It will be appreciated that the port body 1212, or portions thereof, can include a variety of materials, including metals, thermoplastics, ceramics, etc., and can include other joining methods including snap-fitted, adhesive, ultrasonic or other welding, interference fit, etc. as discussed herein. In an example useful for understanding the invention, the port 1210 further includes a portion of the outer shell 1236 that is overmolded onto a portion of the port body 1212. For example, proximal portion 1236A of the outer shell 1236 is formed of a compliant material, such as silicone, or similar suitable material as discussed herein and is overmolded onto the port body 1212.

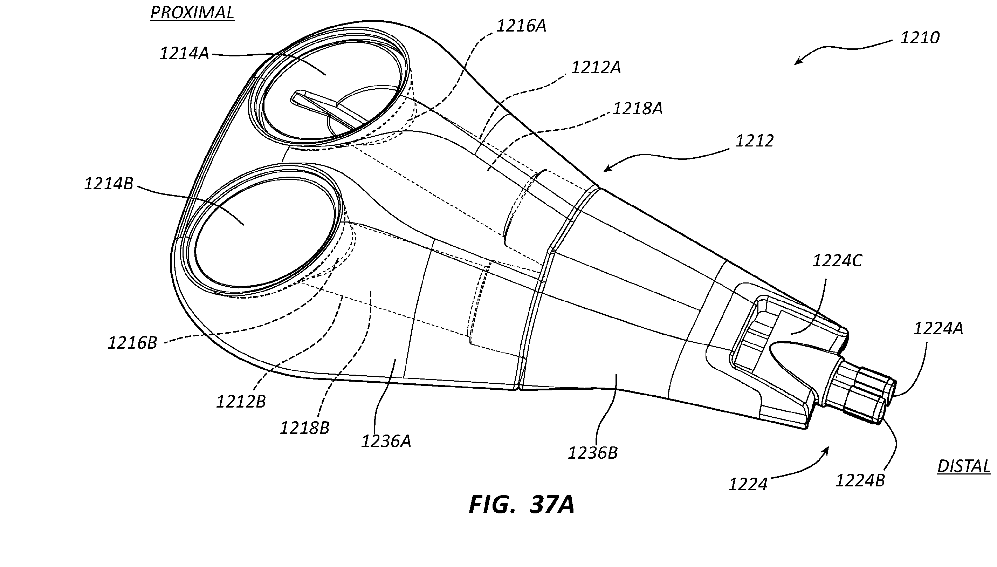

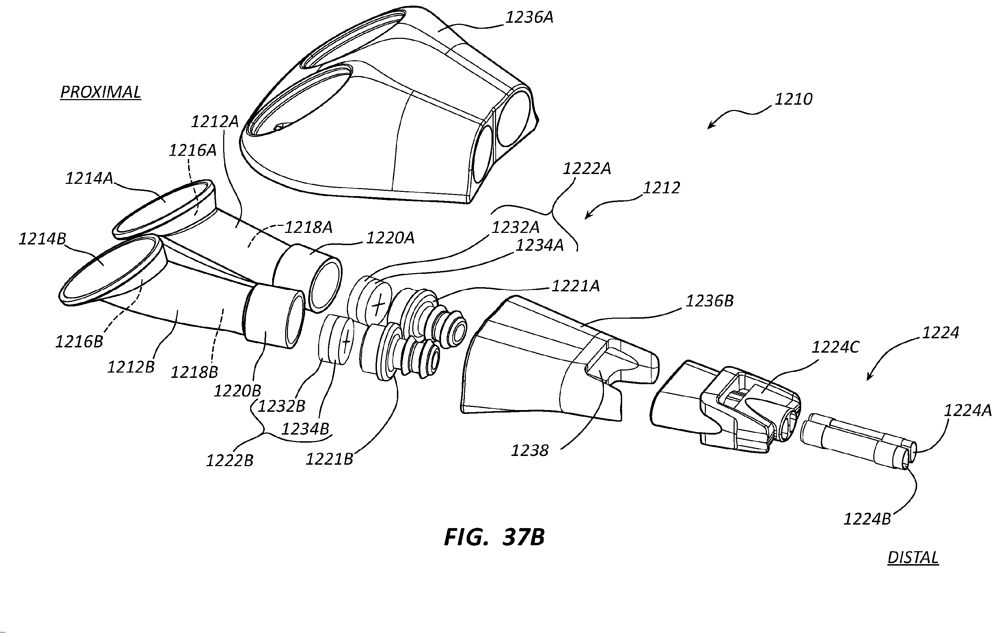

FIGS. 37A-B show further details of the port 1210 of the catheter assembly 1200. Each of the first and second conduits 1212A, 1212B define a substantially funnel-shaped receiving cup 1214 for receiving and directing the catheter-bearing needle 42 (FIG. 14A) to operably connect with the port 1210 in a manner similar to that already described herein. In particular, the substantially funnel shape of each receiving cup 1214 is configured to direct the catheter-bearing needle 42 impinging thereon toward an inlet port 1216 that serves as an opening for a respective conduit 1212. The open and shallow nature of each receiving cup 1214, angled toward the skin surface of the patient enables the receiving cup to present a large, easily accessible target for the needle when introduced into the skin and directed toward the subcutaneously implanted access port 1210.

Each of the first and second conduits 1212A, 1212B further include a valve/seal assembly 1222, such as valve/seal assembly 1222A, 1222B. Each valve/seal assembly 1222 includes a seal 1232 and a valve 1234 disposed in a valve/seal housing 1220. Each valve seal housing 1220 is disposed at a distal end of the respective first and second conduits 1212A, 1212B and secured in place with a nozzle 1221, e.g. nozzle 1221A, B. A distal end of the nozzle 1221 is received within a proximal end of the outer shell distal portion 1236B. Accordingly, the port body 1212, including the respective valve/seal assemblies 1220, nozzles, 1221 and stem 1224 define lumen 1218A, 1218B that extend from an inlet port 1216A, 1216B to a respective outlet of stem 1224A, 1224B. Note that features of other examples described herein, for example palpation features, indicia, septa, guide grooves, valves, seals, etc., can be included with the port 1210.

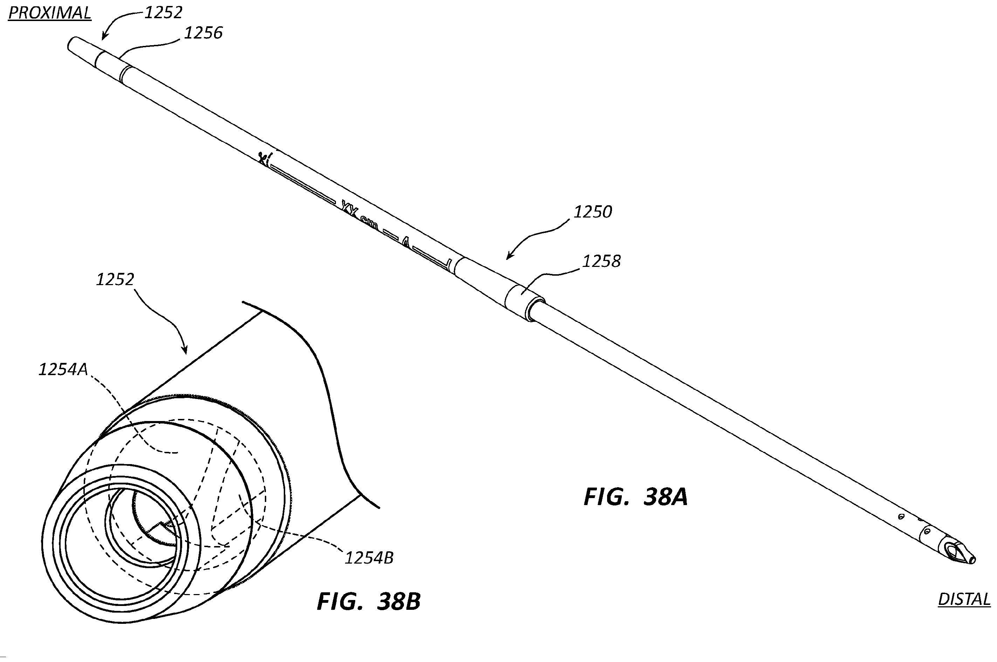

FIG. 38A-38B shows details of an exemplary multi-lumen catheter 1250. The catheter 1250 includes an elongate tube extending from a proximal end to a distal end and can define at least one lumen. Although FIG. 38B shows a dual-lumen catheter it will be appreciated that catheters with greater or fewer lumens are contemplated to fall within the scope of the present invention. A proximal end 1252 is configured to fluidly communicate with stem assembly 1224. In an example, a first stem 1224A communicates with a first lumen 1254A and a second stem 1224B communicates with a second lumen 1254B. -

The catheter 1250 includes an annular collar 1256 disposed proximate a proximal end which co-operates with a locking member 1260, and will be discussed in more detail herein. The catheter 1250 also includes a cuff 1258. The cuff 1258 can be made of, for example DACRON^{™}, or similar suitable material. The cuff 1258 serves as an ingrowth cuff to further secure the catheter upon implantation within the body.

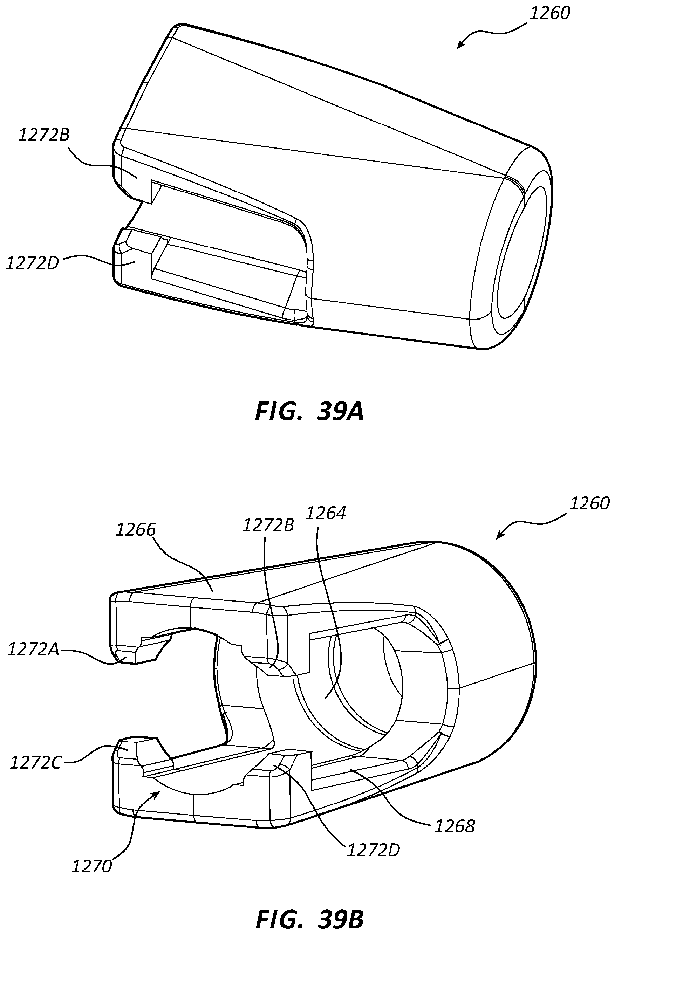

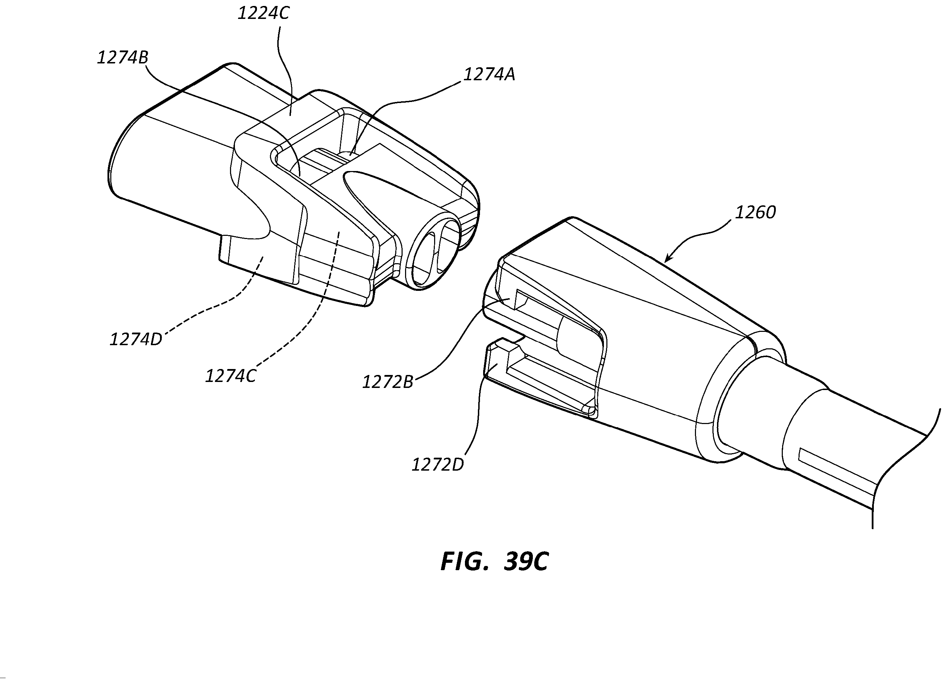

Referring to FIGS. 39A-39C, the catheter assembly 1200 further includes a locking member 1260 that fits over the catheter 1250 and engages the port body 1212, securing the catheter 1250 thereto. To note, FIG. 39C shows the stem assembly housing 1224C, locking member 1260 and catheter 1250, with the first and second stems 1224A, B removed for clarity. The locking member 1260 includes a channel 1262 extending from a proximal end to a distal end of the locking member 1260, and is designed to receive a catheter disposed therethrough. A circumference of the channel 1262 is sized to fit snugly about a circumference of the catheter 1250. The locking member 1260 includes an annular abutment 1264 disposed towards a distal end of the channel 1262 and extending radially inward. The annular abutment 1264 abuts against an annular collar 1256 of the catheter and inhibits longitudinal distal movement of the catheter relative to the locking member 1260.

The locking member 1260 includes an upper and lower portion 1266, 1268 that extend proximally to define an upper and lower surface of the locking member 1260, respectively. The upper and lower portions 1266, 1268 further define openings in the left and rights sides of the locking member 1260. The openings extend proximally, from a distal end, to a point that is proximal of the distal end and are configured to receive a portion of the stem assembly housing 1224C.

The locking member 1260 further includes protrusions 1272 disposed at a distal end of the locking member 1260 and extend transversely inwards. Protrusions 1272A, 1272B extend transversely downwards from a distal end of the upper portion 1266, and protrusions 1272C, 1272D extend transversely upwards from a distal end of the lower portion 1268. The protrusions 1272 co-operate with slots 1274 disposed in an upper and lower surface of the port housing 1224C, such that each protrusion 1272A-D engages a corresponding slot 1274A-D.

The locking member 1260 includes a resilient material such that an upper and lower portions 1266, 1268 are able to flex slightly. Accordingly, as the locking member 1260 is urged distally to engage the housing 1224C the upper and lower portions 1266, 1268 flex outward allowing the housing 1224C to be received within the space defined by the upper and lower portions 1266, 1268 of the connector. Further, the protrusions 1272 can include a chamfer to facilitate sliding over a distal portion of the housing 1224C and engage the slots 1274. Accordingly, the locking member can securely engage the housing 1224C and can align the catheter 1250 with the stem 1224.

Advantageously, the catheter assembly 1200 provides a modular construction where individual components can be press fitted or snap fitted into place, although other methods of attaching are also contemplated. Accordingly, this facilitates manufacture and assembly together with improved associated costs. Moreover, being formed of a modular construction allows individual components to be modified and changed to suit different specifications with minimal interference to the manufacturing process. For example, port body 1212 can be configured to receive different gauge needles, catheters, or the like by exchanging the body conduits 1212A, 1212B, nozzles, 1221, valve assemblies 1222, or the like. Similarly, the catheter 1250 and stem assemblies 1224 can be easily exchanged for catheters of different characteristics such as different gauges, thicknesses, physical characteristics (e.g. materials, durometers), lumens characteristics, tip characteristics, or the like.

The port 1210, locking member 1260 and catheter 1250 can also co-operate to define a substantially smooth outer profile to the catheter system 1200. This advantageously facilitates implantation within a tissue pocket and reduces patient discomfort once implanted. Further, a smooth outer profile allows any palpation features disposed there on to be more pronounced and therefore more easily discernable by a clinician.

The described examples are to be considered in all respects only as illustrative, not restrictive. The scope of the embodiments is, therefore, indicated by the appended claims rather than by the foregoing description.

## Claims

1. A low-profile access port (914), comprising:
a body (912) including:
a first set of receiving cups (914A);
a second set of receiving cups (914B);
a first set of inlet ports, each receiving cup of the first set of receiving cups (914A) in fluid communication with an inlet port of the first set of inlet ports, each receiving cup of the first set of receiving cups (914A) concavely shaped to direct an impinging needle toward a respective inlet port of the first set of inlet ports;
a second set of inlet ports, each receiving cup of the second set of receiving cups (914B) in fluid communication with an inlet port of the second set of inlet ports, each receiving cup of the second set of receiving cups (914B) concavely shaped to direct an impinging needle toward a respective inlet port of the second set of inlet ports; and
a first conduit (918A) in fluid communication with each inlet port of the first set of inlet ports, the first conduit extending from the first set of inlet ports to a first outlet (924A) of a port stem (924);
a second conduit (918B) in fluid communication with each inlet port of the second set of inlet ports, the second conduit extending from the second set of inlet ports to a second outlet of the port stem; and
a catheter in fluid communication with the first outlet.

2. The low-profile access port according to claim 1, wherein the first set of receiving cups is proximal to the second set of receiving cups.

3. The low-profile access port according to any claim of claims 1-2, wherein a perimeter of each receiving cup of the first set of receiving cups lies in a plane (890A; 890B, 990A; 990B), and wherein the plane of the perimeter of each receiving cup is angled with respect to one another.

4. The low-profile access port according to any claim of claims 1-2, wherein a perimeter of each receiving cup of the first set of receiving cups lies in a plane, and wherein the plane of the perimeter of each receiving cup is co-planar with respect to one another.

5. The low-profile access port according to any claim of claims 1-4, wherein a perimeter of each receiving cup of the first set of receiving cups includes a cutout (870, 970), the cutout between adjacent receiving cups providing communication therebetween.

6. A vascular access device (1110) for subcutaneous implantation, comprising:
a catheter having a first lumen and a second lumen;
an elongate body (1112) defining a first elongate chamber (1114A) and a second elongate chamber (1114B), the first elongate chamber in fluid communication with the first lumen and the second elongate chamber in fluid communication with the second lumen;
a needle penetrable septum (1140) disposed over an opening in an upper surface of the elongate body, the opening providing access to the first elongate chamber and the second elongate chamber;
**characterised in that** the vascular access device further comprises
a needle impenetrable needle guide (1142) disposed over the opening and the needle penetrable septum, the needle impenetrable guide including a plurality of first openings (1144) positioned over the first elongate chamber, and a plurality of second openings positioned over the second elongate chamber.

7. The vascular access device according to claim 6, wherein the elongate body has a length and a width, the length more than two times greater than the width.

8. The vascular access device according to any claim of claims 6-7, wherein the first elongate chamber and the second elongate chamber extend in a side-by-side arrangement relative to a longitudinal axis of the elongate body.

9. The vascular access device according to any claim of claims 6-7, wherein the first elongate chamber and the second elongate chamber are in a tandem arrangement relative to a longitudinal axis of the elongate body such that the first elongate chamber is proximal to the second elongate chamber.

10. The vascular access device according to any claim of claims 6-9, wherein the needle impenetrable guide is disposed at least partially within the needle penetrable septum.

11. The vascular access device according to any claim of claims 6-9, wherein the needle impenetrable guide does not penetrate the needle penetrable septum.

12. The vascular access device according to any claim of claims 6-11, wherein the plurality of first openings are parallel to the plurality of second openings.

## Patentansprüche

1. Flacher Zugangsanschluss (914), umfassend:
einen Körper (912),
einschließend:
einen ersten Satz von Aufnahmebechern (914A);
einen zweiten Satz von Aufnahmebechern (914B);
einen ersten Satz von Einlassanschlüssen,
wobei jeder Aufnahmebecher des ersten Satzes von Aufnahmebechern (914A) in Fluidverbindung mit einem Einlassanschluss des ersten Satzes von Einlassanschlüssen steht,
wobei jeder Aufnahmebecher des ersten Satzes von Aufnahmebechern (914A) konkav geformt ist, um eine auftreffende Nadel in Richtung eines entsprechenden Einlassanschlusses des ersten Satzes von Einlassanschlüssen zu lenken,
einen zweiten Satz von Einlassanschlüssen, wobei jeder Aufnahmebecher des zweiten Satzes von Aufnahmebechern (914B) in Fluidverbindung mit einem Einlassanschluss des zweiten Satzes von Einlassanschlüssen steht,
wobei jeder Aufnahmebecher des zweiten Satzes von Aufnahmebechern (914B) konkav geformt ist, um eine auftreffende Nadel in Richtung eines entsprechenden Einlassanschlusses des zweiten Satzes von Einlassanschlüssen zu lenken; und
eine erste Leitung (918A) in Fluidverbindung mit jedem Einlassanschluss des ersten Satzes von Einlassanschlüssen, wobei sich die erste Leitung vom ersten Satz von Einlassanschlüssen zu einem ersten Auslass (924A) eines Anschlussschafts (924) erstreckt;
eine zweite Leitung (918B) in Fluidverbindung mit jedem Einlassanschluss des zweiten Satzes von Einlassanschlüssen, wobei sich die zweite Leitung vom zweiten Satz von Einlassanschlüssen zu einem zweiten Auslass des Anschlussschafts erstreckt; und einen Katheter in Fluidverbindung mit dem ersten Auslass.

2. Flacher Zugangsanschluss nach Anspruch 1, wobei der erste Satz von Aufnahmebechern proximal zum zweiten Satz von Aufnahmebechern ist.

3. Flacher Zugangsanschluss nach einem der Ansprüche 1-2, wobei ein Umfang jedes Aufnahmebechers des ersten Satzes von Aufnahmebechern in einer Ebene (890A; 890B, 990A; 990B) liegt, und wobei die Ebene des Umfangs jedes Aufnahmebechers in Bezug zueinander abgewinkelt ist.

4. Flacher Zugangsanschluss nach einem der Ansprüche 1-2, wobei ein Umfang jedes Aufnahmebechers des ersten Satzes von Aufnahmebechern in einer Ebene liegt, und wobei die Ebene des Umfangs jedes Aufnahmebechers in Bezug zueinander komplanar ist.

5. Flacher Zugangsanschluss nach einem der Ansprüche 1-4, wobei ein Umfang jedes Aufnahmebechers des ersten Satzes von Aufnahmebechern einen Ausschnitt (870, 970) einschließt, wobei der Ausschnitt zwischen benachbarten Aufnahmebechern eine Verbindung dazwischen bereitstellt.

6. Gefäßzugangsvorrichtung (1110) zur subkutanen Implantation, umfassend:
einen Katheter, der ein erstes Lumen und ein zweites Lumen aufweist;
einen länglichen Körper (1112), der eine erste längliche Kammer (1114A) und eine zweite längliche Kammer (1114B) definiert, wobei die erste längliche Kammer in Fluidverbindung mit dem ersten Lumen steht und die zweite längliche Kammer in Fluidverbindung mit dem zweiten Lumen steht;
ein von einer Nadel durchdringbares Septum (1140), das über einer Öffnung in einer oberen Oberfläche des länglichen Körpers angeordnet ist, wobei die Öffnung Zugang zu der ersten länglichen Kammer und der zweiten länglichen Kammer bereitstellt;
**dadurch gekennzeichnet, dass** die Gefäßzugangsvorrichtung weiter
eine von einer Nadel undurchdringbare Nadelführung (1142) umfasst, die über der Öffnung und dem von einer Nadel durchdringbaren Septum angeordnet ist, wobei die von einer Nadel undurchdringbare Führung eine Vielzahl von ersten Öffnungen (1144), die über der ersten länglichen Kammer angeordnet ist, und eine Vielzahl von zweiten Öffnungen, die über der zweiten länglichen Kammer angeordnet ist, einschließt.

7. Gefäßzugangsvorrichtung nach Anspruch 6, wobei der längliche Körper eine Länge und eine Breite aufweist, wobei die Länge mehr als doppelt so groß ist wie die Breite.

8. Gefäßzugangsvorrichtung nach einem der Ansprüche 6-7, wobei sich die erste längliche Kammer und die zweite längliche Kammer in einer nebeneinanderliegenden Anordnung relativ zu einer Längsachse des länglichen Körpers erstrecken.

9. Gefäßzugangsvorrichtung nach einem der Ansprüche 6-7, wobei die erste längliche Kammer und die zweite längliche Kammer in einer Tandemanordnung relativ zu einer Längsachse des länglichen Körpers angeordnet sind, sodass die erste längliche Kammer proximal zur zweiten länglichen Kammer ist.

10. Gefäßzugangsvorrichtung nach einem der Ansprüche 6-9, wobei die von einer Nadel undurchdringbare Führung mindestens teilweise innerhalb des von einer Nadel durchdringbaren Septums angeordnet ist.

11. Gefäßzugangsvorrichtung nach einem der Ansprüche 6-9, wobei die von einer Nadel undurchdringbare Führung das von einer Nadel durchdringbare Septum nicht durchdringt.

12. Gefäßzugangsvorrichtung nach einem der Ansprüche 6-11, wobei die Vielzahl von ersten Öffnungen parallel zu der Vielzahl von zweiten Öffnungen ist.

## Revendications

1. Orifice d'accès à profil bas (914) comprenant :
un corps (912)
incluant :
un premier ensemble de cupules de réception (914A) ;
un second ensemble de cupules de réception (914B) ;
un premier ensemble d'orifices d'entrée,
chaque cupule de réception du premier ensemble de cupules de réception (914A) étant en communication fluidique avec un orifice d'entrée du premier ensemble d'orifices d'entrée,
chaque cupule de réception du premier ensemble de cupules de réception (914A) étant formée de manière concave pour diriger une aiguille incidente vers un orifice d'entrée respectif du premier ensemble d'orifices d'entrée
un second ensemble d'orifices d'entrée, chaque cupule de réception du second ensemble de cupules de réception (914B) étant en communication fluidique avec un orifice d'entrée du second ensemble d'orifices d'entrée, chaque cupule de réception du second ensemble de cupules de réception (914B) étant formée de manière concave pour diriger une aiguille incidente vers un orifice d'entrée respectif du second ensemble d'orifices d'entrée ; et
un premier conduit (918A) en communication fluidique avec chaque orifice d'entrée du premier ensemble d'orifices d'entrée, le premier conduit s'étendant depuis le premier ensemble d'orifices d'entrée jusqu'à une première sortie (924A) d'une tige d'orifice (924) ;
un second conduit (918B) en communication fluidique avec chaque orifice d'entrée du second ensemble d'orifices d'entrée, le second conduit s'étendant depuis le second ensemble d'orifices d'entrée jusqu'à une seconde sortie de la tige d'orifice ; et un cathéter en communication fluidique avec la première sortie.

2. Orifice d'accès à profil bas selon la revendication 1, dans lequel le premier ensemble de cupules de réception est proximal au second ensemble de cupules de réception.

3. Orifice d'accès à profil bas selon une quelconque revendication des revendications 1 à 2, dans lequel un périmètre de chaque cupule de réception du premier ensemble de cupules de réception se situe dans un plan (890A, 890B, 990A, 990B), et dans lequel le plan du périmètre de chaque cupule de réception est incliné par rapport à l'autre.

4. Orifice d'accès à profil bas selon une quelconque revendication des revendications 1 à 2, dans lequel un périmètre de chaque cupule de réception du premier ensemble de cupules de réception se situe dans un plan, et dans lequel le plan du périmètre de chaque cupule de réception est coplanaire par rapport à l'autre.

5. Orifice d'accès à profil bas selon une quelconque revendication des revendications 1 à 4, dans lequel un périmètre de chaque cupule de réception du premier ensemble de cupules de réception inclut une découpe (870, 970), la découpe entre des cupules de réception adjacentes assurant une communication entre celles-ci.

6. Dispositif d'accès vasculaire (1110) pour une implantation sous-cutanée, comprenant :
un cathéter présentant une première lumière et une seconde lumière ;
un corps allongé (1112) définissant une première chambre allongée (1114A) et une seconde chambre allongée (1114B), la première chambre allongée étant en communication fluidique avec la première lumière et la seconde chambre allongée étant en communication fluidique avec la seconde lumière ;
un opercule pénétrable par aiguille (1140) disposé sur une ouverture dans une surface supérieure du corps allongé, l'ouverture donnant accès à la première chambre allongée et à la seconde chambre allongée ;
**caractérisé en ce que** le dispositif d'accès vasculaire comprend en outre
un guide d'aiguille impénétrable par aiguille (1142) disposé sur l'ouverture et l'opercule pénétrable par aiguille, le guide impénétrable par aiguille incluant une pluralité de premières ouvertures (1144) positionnées sur la première chambre allongée, et une pluralité de secondes ouvertures positionnées sur la seconde chambre allongée.

7. Dispositif d'accès vasculaire selon la revendication 6, dans lequel le corps allongé présente une longueur et une largeur, la longueur étant plus de deux fois plus grande que la largeur.

8. Dispositif d'accès vasculaire selon une quelconque revendication des revendications 6 ou 7, dans lequel la première chambre allongée et la seconde chambre allongée s'étendent dans un agencement côte à côte par rapport à un axe longitudinal du corps allongé.

9. Dispositif d'accès vasculaire selon une quelconque revendication des revendications 6 ou 7, dans lequel la première chambre allongée et la seconde chambre allongée sont dans un agencement en tandem par rapport à un axe longitudinal du corps de sorte que la première chambre allongée soit proximale à la seconde chambre allongée.

10. Dispositif d'accès vasculaire selon une quelconque revendication des revendications 6 à 9, dans lequel le guide impénétrable par aiguille est disposé au moins partiellement à l'intérieur de l'opercule pénétrable par aiguille.

11. Dispositif d'accès vasculaire selon une quelconque revendication des revendications 6 à 9, dans lequel le guide impénétrable par aiguille ne pénètre pas l'opercule pénétrable par aiguille.

12. Dispositif d'accès vasculaire selon une quelconque revendication des revendications 6 à 11, dans lequel la pluralité de premières ouvertures sont parallèles à la pluralité de secondes ouvertures.
